(19) Europäisches Patentamt
European Patent Office
Office européen des brevets

(11) **EP 3 367 101 B1**

(12) **EUROPEAN PATENT SPECIFICATION**

(45) Date of publication and mention
of the grant of the patent:
**19.07.2023 Bulletin 2023/29**

(21) Application number: **18158733.8**

(22) Date of filing: **27.02.2018**

(51) International Patent Classification (IPC):
***G01N 33/68*** *(2006.01)*

(52) Cooperative Patent Classification (CPC):
**G01N 33/6893**

(54) **METHOD, APPARATUS AND COMPUTER PROGRAM FOR PREDICTION OF OCCURENCE OF SEVERE ASTHMA ATTACK IN A PATIENT**

VERFAHREN, VORICHTUNG UND COMPUTERPROGRAM ZUR 
VORHERSAGE VON SCHWERE ASTHMAATTACKEN IN EINEM PATEINT

PROCÉDÉ, DISPOSITIF ET PROGRAMME DE PRÉDICTIONTION D'UNE ATTAQUE SEVÈRE D'ASTHME D'UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2017 JP 2017035107**

(43) Date of publication of application:
**29.08.2018 Bulletin 2018/35**

(73) Proprietor: **SYSMEX CORPORATION**
**Kobe-shi**
**Hyogo 651-0073 (JP)**

(72) Inventors:
• **Hasegawa, Takehiro**
  **Kobe-shi, Hyogo, 651-0073 (JP)**
• **Kurata, Hirokazu**
  **Kobe-shi, Hyogo, 651-0073 (JP)**

(74) Representative: **De Clercq & Partners**
**Edgard Gevaertdreef 10a**
**9830 Sint-Martens-Latem (BE)**

(56) References cited:
• JULIE CHESNÉ ET AL: "IL-17 in Severe Asthma. Where Do We Stand?", AMERICAN JOURNAL OF RESPIRATORY AND CRITICAL CARE MEDICINE., vol. 190, no. 10, 15 November 2014 (2014-11-15), pages 1094-1101, XP055465216, US ISSN: 1073-449X, DOI: 10.1164/rccm.201405-0859PP
• AL-RAMLI W ET AL: "TH17-associated cytokines (IL-17A and IL-17F) in severe asthma", JOURNAL OF ALLERGY AND CLINICAL IMMUNOLOGY, ELSEVIER, AMSTERDAM, NL, vol. 123, no. 5, 1 May 2009 (2009-05-01), pages 1185-1187, XP026083387, ISSN: 0091-6749, DOI: 10.1016/J.JACI.2009.02.024 [retrieved on 2009-04-10]
• BULLENS DOMINIQUE MA ET AL: "IL-17 mRNA in sputum of asthmatic patients: linking T cell driven inflammation and granulocytic influx?", RESPIRATORY RESEARCH, BIOMED CENTRAL LTD., LONDON, GB, vol. 7, no. 1, 3 November 2006 (2006-11-03), page 135, XP021021478, ISSN: 1465-9921, DOI: 10.1186/1465-9921-7-135
• NIRAV R. BHAKTA ET AL: "IL-17 and "TH2-high" asthma: Adding fuel to the fire?", JOURNAL OF ALLERGY AND CLINICAL IMMUNOLOGY, vol. 134, no. 5, 1 November 2014 (2014-11-01), pages 1187-1188, XP055465218, AMSTERDAM, NL ISSN: 0091-6749, DOI: 10.1016/j.jaci.2014.07.034
• AGACHE I ET AL: "Increased serum IL-17 is an independent risk factor for severe asthma", RESPIRATORY MEDICINE, BAILLIERE TINDALL, LONDON, GB, vol. 104, no. 8, 1 August 2010 (2010-08-01), pages 1131-1137, XP027106581, ISSN: 0954-6111 [retrieved on 2010-06-26]

EP 3 367 101 B1

- Takehiro Hasegawa ET AL: "Increased serum IL-17A and Th2 cytokine levels in patients with severe uncontrolled asthma", European Cytokine Network, 1 January 2017 (2017-01-01), pages 8-18, XP055465270, Heidelberg DOI: 10.1684/ecn.2017.0390 Retrieved from the Internet: URL:http://www.jle.com/download/ecn-309763 -increased_serum_il_17a_and_th2_cytokine_l evels_in_patients_with_severe_uncontrolled _asthma--Wsd0h38AAQEAAD-wh7MAAAAQ-a.p df [retrieved on 2018-04-07]
- Mark J Fitzgerald: "POCKET GUIDE FOR ASTHMA MANAGEMENT AND PREVENTION A Pocket Guide for Physicians and Nurses Updated 2015 (for Adults and Children Older than 5 Years) BASED ON THE GLOBAL STRATEGY FOR ASTHMA MANAGEMENT AND PREVENTION Global Initiative for Asthma", , 1 December 2015 (2015-12-01), XP055582303, Retrieved from the Internet: URL:https://ginasthma.org/wp-content/uploa ds/2016/01/GINA_Pocket_2015.pdf [retrieved on 2019-04-18]
- Makoto Kudo ET AL: "IL-17A produced by [alpha][beta] T cells drives airway hyper-responsiveness in mice and enhances mouse and human airway smooth muscle contraction", Nature Medicine, vol. 18, no. 4, 1 April 2012 (2012-04-01), pages 547-554, XP055582308, New York ISSN: 1078-8956, DOI: 10.1038/nm.2684

**Description**

TECHNICAL FIELD

The present invention relates to a method, an apparatus and a computer program for distinguishing an asthma patient who is inadequately controlled by medium- to high-dose inhaled corticosteroid therapy and treatment with other asthma drugs, and with high exacerbation risk.

BACKGROUND

Asthma is characterized by chronic inflammation of the respiratory tract and various degrees of reversible airway narrowing and increased airway hyperresponsiveness, and clinically, recurrent cough, wheezing and dyspnea. In the differential diagnosis of asthma, emphasis is placed on (1) repeated paroxysmal cough, wheezing and dyspnea, (2) reversible airflow restriction, and (3) not related to other cardiopulmonary diseases or the like. Treatment of asthma aims to control the occurrence frequency of attacks and ensure a daily life that does not differ from that of healthy person. In the current guidelines, a treatment policy is determined by monitoring asthma symptoms and respiratory function. That is, currently, so-called symptomatic therapy, in which asthma symptoms and respiratory function are monitored to determine a treatment policy, is the mainstream, and an established method for predicting the risk of asthma exacerbation such as the risk of occurrence of a severe attack is not known. Makoto Kuto et al. ("IL-17A produced by $\alpha\beta$ T cells drives airway hyper-responsiveness in mice and enhances muse and human airway smooth muscle contraction" Nature Medicine 18(4):547-554) may suggest suggest a contribution of IL-17 in allergan-induced airway hyper-responsiveness, but is completely silent on the involvement of other cytokines thereon.

SUMMARY OF THE INVENTION

TECHNICAL PROBLEMS

It is known that interleukin 17 (IL-17), a cytokine secreted by Th17 cells, one of helper T cells, shows high levels in sera of severe asthma patients (Ioana Agache et al., "Increased serum IL-17 is an independent risk factor for severe asthma" Respir Med 2010, 104(8): 1131-1137; Chesné et al. "IL-17 ins severe asthma. Where do we stand?" Am. J. Resp. Crit. Care Med. 2014, 190(10):1094-1101; Al-Ramli et al. "TH17-associated cytokines (IL-17A and IL-17F) in severe asthma" J. Allergy Clin. Immunol. 2009,123(5):1185-1187; Bullens et al. "IL-17 mRNA in sputum of asthmatic patients: linking T cell driven inflammation and granulocyte influx?" Respir Res, 2006, 7(1):135; Nirav Bhakta et al. "IL-17 and TH2-high ashtrma: Adding fuel to the fire?" J. Allergy Clin. Immunol. 2014, 134(5):1187-1188; Hasegawa et al. "Increased serum IL-17A and Th2 cytokine levels in patients with severe uncontrolled asthma" Eur Cytokine Netw 2017, 28(1):8-18). However, elevated levels of IL-17 also occur in mild and moderate asthma patients, and there was no known relation between the concentration of IL-17 in the serum and the exacerbation risk such as the possibility of occurrence of a future severe attack. Although IL-17 is a member of the IL-17 family including IL-17A, IL-17B and the like, the one used as a biomarker in the present invention is IL-17A. Hereinafter, IL-17A may be simply referred to as "IL-17".

Although the terms "risk factor" and "predictor" are used in Ioana Agache et al. (2010, above), they are used in a meaning different from the exacerbation risk (prediction of the possibility of future exacerbation) referred in the present invention. That is, Ioana Agache et al. (2010, above) has reported that asthma patients are classified into severe, moderate and mild, in accordance with the Global Initiative for Asthma (GINA) asthma guidelines, and the serum levels of IL-17 are significantly higher in patients classified as severe in this classification. Based on this result, these terms are used as indications as to whether patients can be diagnosed as severe asthma only on the basis of the blood IL-17 concentration. The diagnostic criteria for diagnosing a patient as severe in the GINA guideline is based on whether or not the symptoms of asthma can be controlled even by a high-capacity inhaled corticosteroid (ICS). Whether or not severe attacks as described in the present invention will occur even in patients diagnosed as severe in the same guideline has not been made clear.

An object of the present invention is to acquire information on the asthma exacerbation risk and contribute to the determination of a patient's treatment policy based on the information.

SOLUTIONS TO PROBLEMS

The present inventors have examined the concentrations of various cytokines in sera of asthma patients and clinical data of the patients and consequently found that there is a significant correlation between the concentration of IL-17 and the subsequent exacerbation history of the patient, and the present invention is based thereon. In addition, the present inventors have found that a group of patients with high exacerbation risk can be distinguished by combining the concentration of IL-17 with the concentration of other interleukins.

More specifically, according to the present invention, there is provided a method for distinguishing an asthma patient who is inadequately controlled by medium- to high-dose inhaled corticosteroid therapy and treatment with other asthma drugs, and with high exacerbation risk, the method comprising measuring a biomarker in a blood sample of an asthma patient and determining drug response and exacerbation risk in the asthma patient by comparing the measured levels of the biomarkers acquired in the measurement step with a predetermined threshold level corresponding to each biomarker, wherein it is determined that the exacerbation risk in the asthma patient is high, and the drug response is low when the measured level of IL-17 is equal to or greater than the predetermined threshold level and the measured level of any one of IL-10, IL-4, IL-25, IL-9 and IFN-$\gamma$ is equal to or larger than the corresponding predetermined threshold level,

wherein the drug response is the response to medium-to high dose inhaled corticosteroid therapy and treatment with other asthma drugs, and wherein the biomarker comprises IL-17 and at least one selected from the group consisting of IL-10, IL-4, IL-25, IL-9 and IFN-$\gamma$.

In addition, according to the present invention, there is provided a method for distinguishing an asthma patient who is inadequately controlled by medium- to high-dose inhaled corticosteroid therapy and treatment with other asthma drugs, and with high exacerbation risk, the method comprising measuring IL-17 and at least one other cytokine biomarker selected from the group consisting of IL-10 (interleukin 10), IL-4 (interleukin 4), IL-25 (interleukin 25), IL-9 (interleukin 9) and IFN-$\gamma$ (interferon gamma) in a blood sample of an asthma patient to acquire a parameter set of biomarkers, classifying the asthma patient into the group with the highest correlation by performing cluster analysis using the parameter set of biomarkers, a first parameter set and a second parameter set, and determining the exacerbation risk in the asthma patient and the drug response wherein it is determined that the asthma patient has a high exacerbation risk and a low drug response when classified into the same group as the first parameter set, and

wherein it is determined that the asthma patient has a high drug response while having a high exacerbation risk, or that the asthma patient has a low exacerbation risk when classified into the same group as the second parameter set, wherein the drug response is the response to medium-to high dose inhaled corticosteroid therapy and treatment with other asthma drugs, in which the parameter set of biomarkers contains the measured level of IL-17 and the measured level of at least one biomarker selected from the group consisting of IL-10, IL-4, IL-25, IL-9 and IFN-$\gamma$, the first parameter set contains the measured level of IL-17 and the measured level of the at least one biomarker selected from the group consisting of IL-10, IL-4, IL-25, IL-9 and IFN-$\gamma$ in a blood sample of an asthma patient previously known to have an occurrence history of exacerbations and have low response to medium- to high-dose inhaled corticosteroid therapy and treatment with other asthma drugs, , and the second parameter set contains the measured level of IL-17 and the measured level of the at least one biomarker selected from the group consisting of IL-10, IL-4, IL-25, IL-9 and IFN-$\gamma$ in a blood sample of an asthma patient previously known to have high response to medium- to high-dose inhaled corticosteroid therapy and treatment with other asthma drugs while having an occurrence history of exacerbations, or have no occurrence history of exacerbations.

According to the present invention, there is provided an apparatus for distinguishing an asthma patient who is inadequately controlled by medium- to high-dose inhaled corticosteroid therapy and treatment with other asthma drugs, and with high exacerbation risk, the apparatus comprising a measurement device for measuring a biomarker in a blood sample of an asthma patient and a determination device connected to the measurement device, wherein the determination device comprises a computer containing a processor and a memory under control of the processor, in which the memory stores a computer program for making the apparatus execute the method of the claimed invention.

According to the present invention, there is provided an apparatus for distinguishing an asthma patient who is inadequately controlled by medium- to high-dose inhaled corticosteroid therapy and treatment with other asthma drugs, and with high exacerbation risk, the apparatus comprising a measurement device for measuring a biomarker in a blood sample of an asthma patient and a determination device connected to the measurement device,

wherein the determination device comprises a computer containing a processor and a memory under control of the processor, in which the memory stores a computer program for making the apparatus execute the method of the claimed invention, wherein the memory stores the first parameter set and the second parameter set, wherein the processor receives the parameter set of biomarkers measured in the measurement step, and reads the first parameter set and the second parameter set from the memory. The asthma patient is classified into a group, based on the highest correlation with the first or second parameter set, in the determination step. When classified into the same group as the first parameter set, the asthma patient is determined to have a high exacerbation risk and have low drug response. When classified into the same group as the second parameter set, the asthma patient is determined to have high drug response while having a high exacerbation risk, or have a low exacerbation risk. The parameter set of biomarkers contains the measured level of IL-17 and the measured level of at least one biomarker selected from the group consisting of IL-10, IL-4, IL-25, IL-9 and IFN-$\gamma$, the first parameter set contains the measured level of IL-17 in a blood sample of an asthma patient previously known to have an occurrence history of exacerbations and have low response to medium- to high-dose inhaled corticosteroid therapy and treatment with other asthma drugs, and the measured level of the at least one biomarker selected from the group consisting of IL-10, IL-4, IL-25, IL-9 and IFN-γ in the blood sample of the asthma patient, and the second parameter set contains the measured level of IL-17 in a blood sample of an asthma patient previously known to have high response to medium- to high-dose inhaled corticosteroid therapy and treatment with other asthma drugs while having an occurrence history of exacerbations, or to have no occurrence history of exacerabations, and the measured level of the at least one biomarker selected from the group consisting of IL-10, IL-4, IL-25, IL-9 and IFN-γ in the blood sample of the asthma patient.

According to the present invention, there is provided a computer program for distinguishing an asthma patient who is inadequately controlled by medium- to high-dose inhaled corticosteroid therapy and treatment with other asthma drugs, and with high exacerbation risk, which is recorded on a computer readable medium, the computer program comprising instructions to make the apparatus of the claimed invention execute the method of the claimed invention.

ADVANTAGEOUS EFFECTS OF INVENTION

According to the present invention, it is possible to provide a method for distinguishing an asthma patient who is inadequately controlled by medium- to high-dose inhaled corticosteroid therapy and treatment with other asthma drugs, and with high exacerbation risk.

BRIEF DESCRIPTION OF THE DRAWINGS

Fig. 1A is an example of the external appearance of an example of a reagent kit disclosed herein but not covered by the claims .

Fig. 1B is an example of the external appearance of example of a reagent kit disclosed herein but not covered by the claims.

Fig. 1C is an example of the external appearance of a reagent kit disclosed herein but not covered by the claims.

Fig. 2 is a schematic diagram showing an example of an apparatus.

Fig. 3 is a block diagram showing a functional configuration of the apparatus in Fig. 2.

Fig. 4 is a block diagram showing a hardware configuration of the apparatus shown in Fig. 2.

Fig. 5A is an example of a flowchart of determination for acquiring data to assist prediction of the possibility of occurrence of a severe attack of an asthma patient, using the apparatus shown in Fig. 2.

Fig. 5B is an example of a flowchart of determination for acquiring data to assist prediction of the possibility of occurrence of a severe attack of an asthma patient, using the apparatus shown in Fig. 2.

Fig. 5C is an example of a flowchart of determination for acquiring data to assist prediction of the possibility of occurrence of a severe attack of an asthma patient, using the apparatus shown in Fig. 2.

Fig. 6A is an example of determination by a method disclosed herein but not covered by the claims.

Fig. 6B is an ROC curve in a case in Example 1.

Fig. 7 is a diagram showing results of cluster analysis in Example 3.

Fig. 8A is a graph showing treatment steps of asthma patients to be analyzed in Example 3.

Fig. 8B is a graph showing control status of asthma patients to be analyzed in Example 3.

Fig. 8C is a graph showing the presence or absence of a history of a severe attack within 12 months of asthma patients to be analyzed in Example 3.

Fig. 9A is a diagram showing results of cluster analysis performed using IL-17 and IL-25 in Example 4.

Fig. 9B is a diagram showing results of cluster analysis performed using IL-17 and IL-4 in Example 4.

Fig. 9C is a diagram showing results of cluster analysis performed using IL-17 and IL-9 in Example 4.

Fig. 9D is a diagram showing results of cluster analysis performed using IL-17 and IL-10 in Example 4.

Fig. 9E is a diagram showing results of cluster analysis performed using IL-17 and IFN-γ in Example 4.

DETAILED DESCRIPTION OF THE PREFERRED EMBODIMENTS

A method for distinguishing an asthma patient who is inadequately controlled by medium-to high-dose inhaled corticosteroid therapy and treatment with other asthma drugs, and with high exacerbation risk of the present invention includes a step of measuring a biomarker in a blood sample of an asthma patient.

Asthma patients are, for example, patients diagnosed as suffering from asthma by a doctor, adults or children who may be suffering from asthma, and the like.

Blood samples are blood (whole blood), serum or plasma. Especially preferred are blood samples that are easy to collect, for example, peripheral blood, and serum and plasma acquired therefrom, and the like.

The biomarker includes IL-17. IL-17 is known as a cytokine that causes Th17 inflammation associated with autoimmune diseases and bacterial inflammatory responses, induces IL-8, IL-6 and causes neutrophilic inflammation. In the present invention, it is possible to provide doctors with data to distinguish an asthma patient who is inadequately controlled by medium- to high-dose inhaled corticosteroid therapy and treatment with other asthma drugs, and with high exacerbation risk, by measuring IL-17 in a blood sample of an asthma patient. This is revolutionary in that it can provide a new and established method capable of predicting beforehand the asthma exacerbation risk such as the risk of occurrence of a severe attack, and allow a different basis for asthma treatment, for which presently so-called symptomatic therapy is mainstream, in which asthma symptoms and respiratory function are monitored to determine a treatment policy.

The biomarker further includes at least one other cytokine selected from the group consisting of IL-10, IL-4, IL-25, IL-9 and IFN-γ. IL-10 is a cytokine produced from antiinflammatory cells such as Treg, MSC and M2 macrophages. IL-10 is known as an antiinflammatory cytokine. IL-4 is known as a Th2 cytokine that induces allergic immune responses such as atopy. IL-25 is known as a ProTh2 cytokine that causes Th2 inflammation. IL-9 is known as a cytokine secreted from Th2 immune cells. IL-9 proliferates mast cells and cells responsible for Th2 immune responses. IFN-γ is known as a Th1 cytokine that responds to viral infections and the like. IFN-γ is secreted from Th1 cells, cytotoxic CD8 cells and the like. IFN-γ activates macrophages and the like. Also, IFN-γ suppresses Th2 immune responses. The biomarkers used consist of IL-17 and one more other cytokine biomarker selected from this group. By measuring at least one biomarker selected from the group consisting of IL-10, IL-4, IL-25, IL-9 and IFN-γ, in addition to IL-17, it is possible to enhance determination accuracy on the exacerbation risk, or provide doctors with information on whether or not the drug response is high, among the asthma patients determined to have a high exacerbation risk due to high measured level of IL-17. The phrase "drug response is high" means that agents prescribed for asthma treatment such as inhaled steroids, leukotriene receptor antagonists (LTRA), long-acting $\beta_2$ stimulants (LABA) and the like relatively easily work. For example, asthma patients with high drug response do not have a severe attack even when they receive treatment of Step 3 or 4 prescribed in Asthma Prevention/Management Guideline 2009. On the other hand, the phrase "drug response is low" means that agents prescribed for asthma treatment relatively hardly work. For example, asthma patients with low drug response have a severe attack even when they receive treatment of Step 3 or 4 prescribed in Asthma Prevention/Management Guideline 2009.

The method for measuring a biomarker is not particularly limited as long as it is a measurement method based on an antigen-antibody reaction well known to those skilled in the art. Examples thereof include ELISA method, western blot method and the like, and ELISA method is preferably used. The antibody used in these measurement methods is not particularly limited as long as it is an antibody capable of specifically recognizing and binding each biomarker in a blood sample. The antibody can be a monoclonal or polyclonal full-length antibody or antibody fragment (Fab, F(ab')$^2$), or the like. The method for preparing such antibody is known to those skilled in the art. Alternatively, the antibody may be a commercially available product. For example, a commercially available protein quantification kit known to those skilled in the art, such as Human IL-17 Quantikine ELISA Kit manufactured by R&D Systems, LEGEND MAX (trademark) Human IL-17A ELISA Kit manufactured by BioLegend Inc. or the like may be used. As an example of the measurement step, the case of measuring a biomarker by a sandwich ELISA method will be described below.

First, a complex containing a biomarker in a blood sample, an antibody for capturing the biomarker (hereinafter also referred to as "capture antibody") and an antibody for detecting the biomarker (hereinafter also referred to as "detection antibody") is formed on a solid phase. This complex can be formed by mixing a blood sample, a capture antibody, and a detection antibody. Then, a solution containing the complex is brought into contact with a solid phase capable of capturing the capture antibody, whereby the complex can be formed on the solid phase. Alternatively, a solid phase preliminarily immobilized with the capture antibody may be used. That is, a solid phase immobilized with the capture antibody, the blood sample, and the detection antibody are brought into contact with each other, whereby the complex can be formed on the solid phase. When both the capture antibody and the detection antibody are monoclonal antibodies, it is preferable that the epitopes be different from each other.

The mode of immobilization of the capture antibody on the solid phase is not particularly limited. For example, the capture antibody and the solid phase may be bound directly, or the capture antibody and the solid phase may be indirectly bound via another substance. Examples of the direct bond include physical adsorption and the like. Examples of the indirect bond include a bond via a combination of biotin and avidin or streptavidin (hereinafter, also referred to as "avidins"). In this case, by preliminarily modifying the capture antibody with biotin and previously binding avidins to the solid phase, the capture antibody and the solid phase can be indirectly bound via the bond between the biotin and the avidins.

The material of the solid phase is not particularly limited, and it can be selected from, for example, organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, etc.), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include particles, membranes, microplates, microtubes, test tubes, and the like.

A biomarker can be measured by detecting the complex formed on the solid phase by a method known in the art. For example, when an antibody labeled with a labeling substance is used as a detection antibody, a biomarker can be measured by detecting a signal generated by the labeling substance. Alternatively, also when a labeled secondary antibody against the detection antibody is used, the biomarker can be measured in the same manner.

In the present methods, B/F (Bound/Free) separation for removing an unreacted free component not forming a complex may be performed between the process of forming the complex and the process of detecting the complex. The unreacted free component refers to a component not constituting a complex. Examples thereof include capture antibodies not bound to the biomarker, detection antibodies, and the like. The means of B/F separation is not particularly limited, and when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particles with a magnet. After removing the unreacted free component, the solid phase capturing the complex may be washed with a suitable aqueous medium such as PBS.

The phrase "detecting a signal" herein includes qualitatively detecting the presence or absence of a signal, quantifying a signal intensity, and semi-quantitatively detecting the intensity of a signal. Semi-quantitative detection means to show the intensity of the signal in stages such as "no signal generated", "weak", "medium", "strong", and the like. In the present methods, it is preferable to detect the intensity of a signal quantitatively or semi-quantitatively.

The labeling substance is not particularly limited as long as a detectable signal is generated. For example, it may be a substance which itself generates a signal (hereinafter, also referred to as "signal generating substance") or a substance which catalyzes the reaction of other substances to generate a signal. Examples of the signal generating substance include fluorescent substances, radioactive isotopes, and the like. Examples of the substance that catalyzes the reaction of other substances to generate a detectable signal include enzymes and the like. Examples of the enzymes include alkaline phosphatase, peroxidase, β-galactosidase, luciferase, and the like. Examples of the fluorescent substances include fluorescent dyes such as fluorescein isothiocyanate (FITC), rhodamine and Alexa Fluor (registered trademark), fluorescent proteins such as GFP, and the like. Examples of the radioisotopes include $^{125}$I, $^{14}$C, $^{32}$P, and the like. Among them, an enzyme is preferable as a labeling substance, and alkaline phosphatase and peroxidase are particularly preferable.

Methods for detecting a signal themselves are known in the art. In the present methods, a measurement method according to the type of signal derived from the labeling substance may be appropriately selected. For example, when the labeling substance is an enzyme, signals such as light and color generated by reacting a substrate for the enzyme can be measured by using a known apparatus such as a spectrophotometer.

The substrate of the enzyme can be appropriately selected from known substrates according to the type of the enzyme. For example, when alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate. Also, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB).

When the labeling substance is a radioactive isotope, radiation as a signal can be measured using a known apparatus such as a scintillation counter. In addition, when the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used.

The detection result of the signal may be used as the measured level of the biomarker. For example, when quantitatively detecting the intensity of a signal, the signal intensity value itself or a value acquired from the intensity value can be used as the measured level of the biomarker. Examples of the value acquired from the signal intensity value include a value obtained by subtracting the signal intensity value of a negative control sample or a background value from the signal intensity value of the biomarker. The negative control sample can be appropriately selected, and examples thereof include urine obtained from a healthy subject and the like. The measured level of the biomarker can be expressed in any form or unit such as concentration, intensity, level, mass (weight) or ratio.

In the present methods, measurement data of the biomarker may be acquired for a plurality of standard samples of known biomarker concentration, and a calibration curve showing the relationship between the biomarker concentration and the measurement data of the biomarker may be prepared. The measurement data of the biomarker acquired from a blood sample can be applied to this calibration curve to acquire the value of the biomarker concentration in the blood sample and used as the measured level of the biomarker.

In the present methods, the biomarker in the blood sample may be measured by a sandwich ELISA method using a capture antibody immobilized on magnetic particles and a detection antibody labeled with a labeling substance. In this case, measurement may be carried out using a commercially available fully automated immunoassay system such as HISCL series (manufactured by Sysmex Corporation).

The method of the present invention includes a step of determining drug response and exacerbation risk based on the measured level of the biomarker obtained as described above.

The exacerbation risk of an asthma patient is preferably a risk of an exacerbation occurring in an asthma patient within 12 months.

The predetermined threshold level is not particularly limited. The predetermined threshold level can be appropriately set. For example, the predetermined threshold level may be set as follows. First, blood is sampled from a plurality of asthma patients, and the biomarker concentration in the blood samples is measured. Data of the measured biomarker concentration is classified into data of asthma patient group having an occurrence history of exacerbations and data of asthma patient group having no occurrence history of exacerbations. The phrase "having an occurrence history of a severe attack" means that an asthma patient has had a severe attack in the past. Then, a value that can most accurately distinguish the biomarker concentration in the blood samples of the asthma patient group having an occurrence history of exacerbations from the biomarker concentration in the blood samples of the asthma patient group having no occurrence history of exacerbations, and the value is set as the predetermined threshold level. In setting the threshold level, it is preferable to consider sensitivity, specificity, positive predictive value, negative predictive value, and the like.

In addition to the measured level of IL-17, the measured level of at least one biomarker selected from the group consisting of IL-10, IL-4, IL-25, IL-9 and IFN-$\gamma$ is further used. By using a plurality of biomarkers, it is possible to enhance accuracy of determination and further determine drug response.

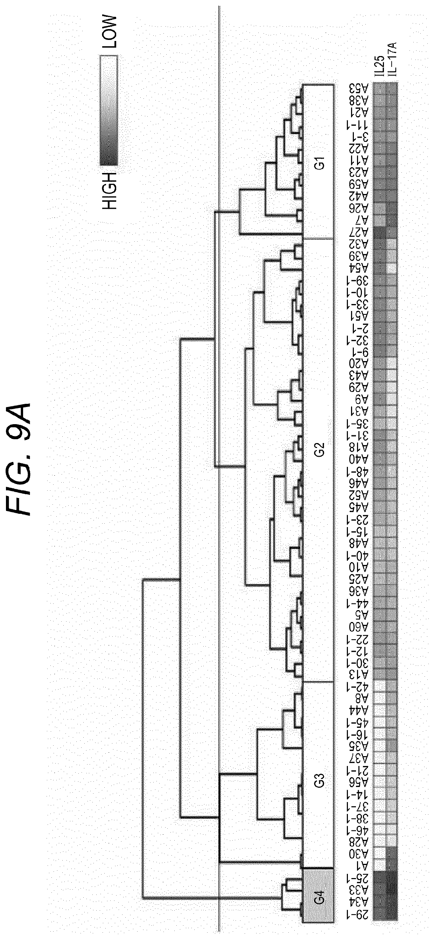

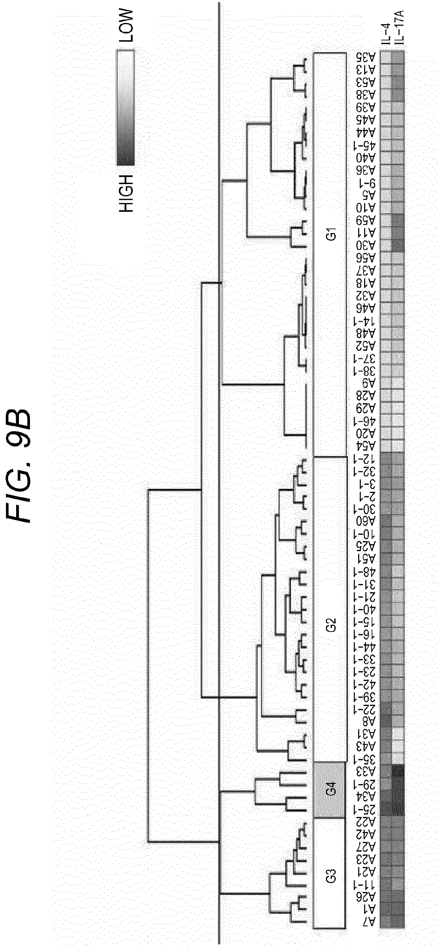

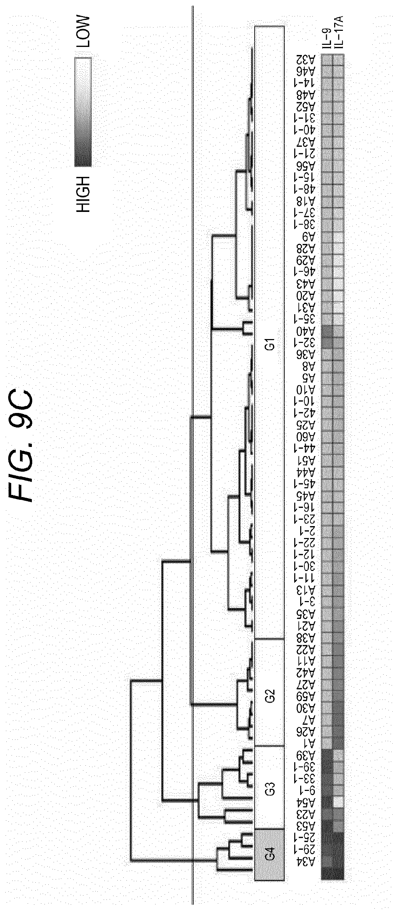

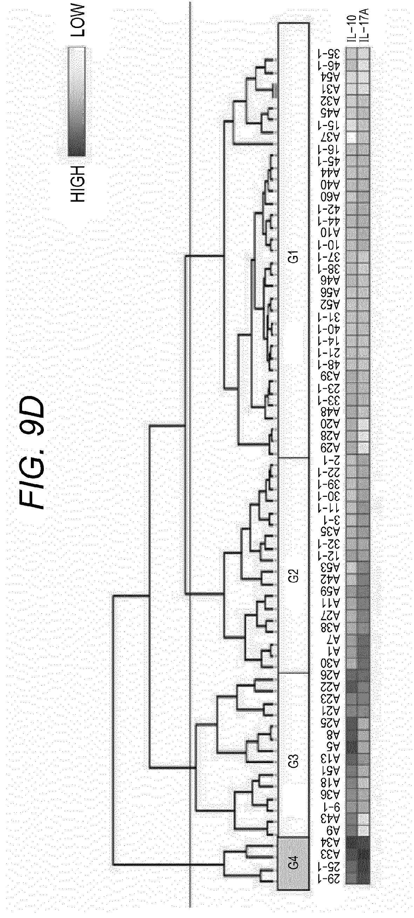

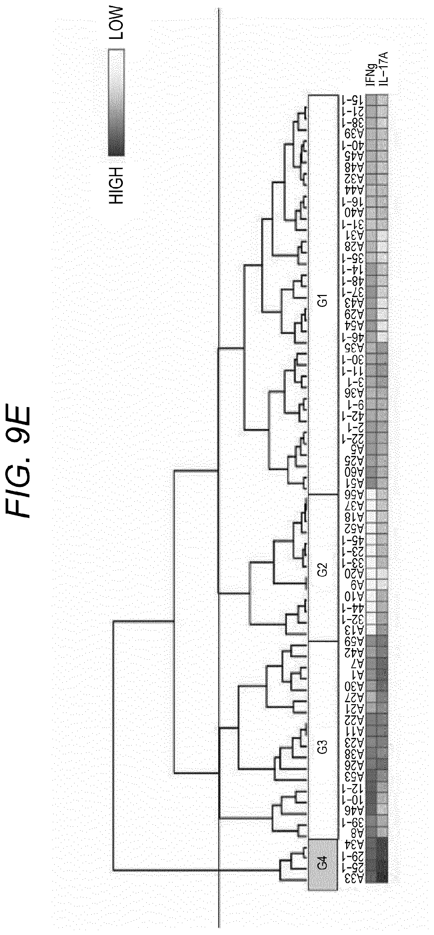

The measured level of each biomarker is compared with the predetermined threshold level corresponding to each, and a determination is made based on the comparison result. More specifically, when the measured level of IL-17 is equal to or greater than the predetermined threshold level and the measured level of any any one of IL-10, IL-4, IL-25, IL-9 and IFN-$\gamma$ is equal to or larger than the corresponding predetermined threshold level, it can be determined that the exacerbation risk in the asthma patient is high, and the drug response is low. Such determination is supported by Figs. 9A to 9E. For example, in the determination of Fig. 9A, in a group of patients (G4) whose measured level of IL-17 is higher than a predetermined threshold level corresponding to IL-17 and whose measured level of IL-25 is higher than a predetermined threshold level corresponding to IL-25, there are many patients with a history of a severe attack.

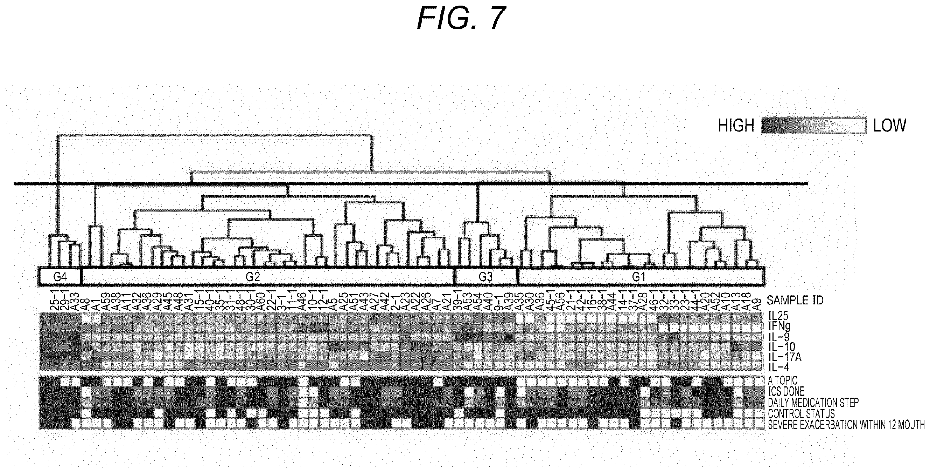

On the other hand, when the measured level of IL-17 is equal to or greater than the predetermined threshold level and all the measured levels of IL-10, IL-4, IL-25, IL-9 and IFN-$\gamma$ are less than the corresponding predetermined threshold level, or when the measured level of IL-17 is less than the predetermined threshold level, it can be determined that the drug response is high while the exacerbation risk in the asthma patient is high, or the exacerbation risk in the asthma patient is low. Such determination is supported by Fig. 7.

In another example disclosed herein but not covered by the claims, a determination is made using multivariate analysis like multiple logistic analysis (logistic regression analysis). In such multivariate analysis, in addition to the measured level of IL-17, the measured level of at least one biomarker selected from the group consisting of IL-10, IL-4, IL-25, IL-9 and IFN-$\gamma$ are substituted into discriminant prepared by multivariate analysis, for example, multiple logistic analysis, to acquire a value acquired from the measured level of the biomarker by multivariate analysis, for example, a predicted value, and in the determination step, a value acquired from the measured level of the biomarker by multivariate analysis is compared with the predetermined threshold level corresponding to a value acquired by multivariate analysis, and the possibility of occurrence of a severe attack of the asthma patient is determined based on the comparison result.

An example of determination using multiple logistic analysis as multivariate analysis will be more specifically described. For example, when the measured levels of IL-17 and IL-10 are acquired, the predicted value may be calculated using a regression equation of the following formula (I). Also, when the measured levels of IL-17, IL-10 and IL-4 are acquired, the predicted value may be calculated using a regression equation of the following formula (II). These regression equations are equations built based on the multiple logistic model. In the formulae, [IL-17], [IL-10] and [IL-4] are measured levels of IL-17, IL-10 and IL-4, respectively, $b_0$ is a coefficient of the intercept, and $b_1$ to $b_3$ are coefficients of each free protein biomarker. The coefficients can be determined by a known method such as a maximum likelihood method.

[Expression 1]

$$P = \frac{1}{1 + \exp[-(b_0 + b_1 \times [\text{IL-17}] + b_2 \times [\text{IL-10}])]}$$

[Expression 2]

$$P = \frac{1}{1 + \exp[-(b_0 + b_1 \times [\text{IL-17}] + b_2 \times [\text{IL-10}] + b_3 \times [\text{IL-4}])]}$$

The predetermined threshold level corresponding to each of the above biomarkers and the threshold level corresponding to the predicted value are not particularly limited and can be set empirically, for example, by accumulating biomarker data in blood samples collected from asthma patients. Alternatively, predetermined cutoff values may be set as follows. Blood samples are collected from a healthy person and an asthma patient, and measured levels of biomarkers are acquired. Then, from the acquired measured levels, values that can distinguish between the healthy person and the asthma patient for each biomarker are obtained, and the obtained values are set as predetermined cutoff values corresponding to each biomarker. Alternatively, the measured levels of biomarkers acquired from liquid samples of a healthy person and an asthma patient are substituted into discriminant prepared by multivariate analysis to acquire a predicted value of the healthy person and a predicted value of the asthma patient. Then, based on the acquired predicted values, values that can distinguish between the healthy person and the asthma patient are obtained, and the obtained values are set as predetermined cutoff values corresponding to the predicted values.

In another embodiment, a determination is made using cluster analysis. In this embodiment, a step of measuring IL-17 and at least one biomarker selected from the group consisting of IL-10, IL-4, IL-25, IL-9 and IFN-$\gamma$ in a blood sample of an asthma patient and acquiring a parameter set of biomarkers is included. The "parameter set of biomarkers" herein means a group of measured levels of a plurality of types of biomarkers obtained for one asthma patient, including the measured level of IL-17 and the measured level of at least one biomarker selected from the group consisting of IL-10, IL-4, IL-25, IL-9 and IFN-$\gamma$. For example, when measuring IL-17, IL-10, IL-25 and IFN-$\gamma$ in a blood sample of an asthma patient, the parameter set of biomarkers means the measured level of IL-17, the measured level of IL-10, the measured level of IL-25 and the measured level of IFN-$\gamma$ in the blood sample of the asthma patient.

As a second step, a step of classifying the asthma patient into the group with the highest correlation by performing cluster analysis using the parameter set of biomarkers obtained in the above measurement step, a first parameter set and a second parameter set is included. The terms "classifying the asthma patient into the group with the highest correlation" as used herein refers to determining whether there is a higher correlation of the values in the parameter set of biomarkers with the first parameter set or the second parameter set.

The cluster analysis is preferably hierarchical cluster analysis. The group with the highest correlation is preferably determined using the Euclidean distance obtained by the nearest neighbor method.

The first parameter set contains the measured level of IL-17 in a blood sample of an asthma patient previously known to have an occurrence history of exacerbations and have low response to medium- to high-dose inhaled corticosteroid therapy and treatment with other asthma drugs, and the measured level of the at least one biomarker selected from the group consisting of IL-10, IL-4, IL-25, IL-9 and IFN-$\gamma$ in the blood sample of the asthma patient . The second parameter set contains the measured level of IL-17 in a blood sample of an asthma patient previously known to have high response to medium- to high-dose inhaled corticosteroid therapy and treatment with other asthma drugs while having an occurrence history of exacerabations, or have no occurrence history of exacerabtions, and the measured level of the at least one biomarker selected from the group consisting of IL-10, IL-4, IL-25, IL-9 and IFN-$\gamma$ in the blood sample of the asthma patient.

For example, when the parameter set of biomarkers contains the measured level of IL-17, the measured level of IL-10, the measured level of IL-25 and the measured level of IFN-$\gamma$ in a blood sample of an asthma patient, the first parameter set contains the measured level of IL-17, the measured level of IL-10, the measured level of IL-25 and the measured level of IFN-$\gamma$ in a blood sample of an asthma patient previously known to have an occurrence history of exacerbations and have low response to medium- to high-dose inhaled corticosteroid therapy and treatment with other asthma drugs, and the second parameter set contains the measured level of IL-17, the measured level of IL-10, the measured level of IL-25 and the measured level of IFN-$\gamma$ in a blood sample of an asthma patient previously known to have high response to medium- to high-dose inhaled corticosteroid therapy and treatment with other asthma drugs while having an occurrence history of exacerbations, or have no occurrence history of exacerbations.

Classification by cluster analysis is preferably performed by comparing the parameter set of biomarkers, the first parameter set, and the second parameter set.

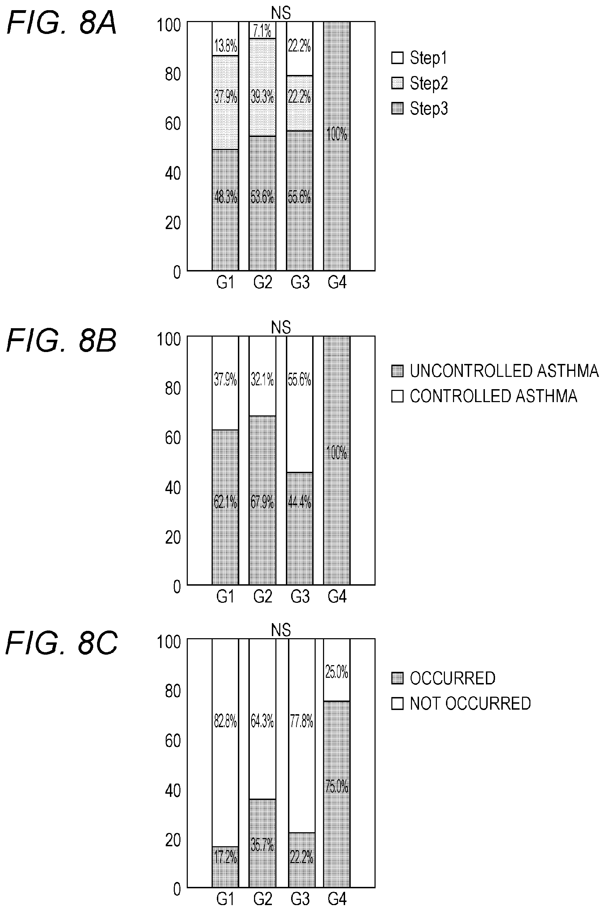

The method provided herein using cluster analysis for determination includes a third step of determining the exacerbation risk in the asthma patient and the drug response based on the result obtained in the above classification step: when classified into the same group as the first parameter set, the asthma patient is determined to have a high exacerbation risk and have low drug response, and when classified into the same group as the second parameter set, the asthma patient is determined to have high drug response while having a high exacerbation risk, or have a low exacerbation risk. Such determination is supported by Figs. 8A and 8C. That is, as shown in Figs. 8A and 8C, in a cluster (G4) which has a high possibility of occurrence of a severe attack and has low drug response, many patients have a severe attack within 12 months even when they have received treatment of Step 3 shown in Table 3. On the other hand, as shown in Figs. 8A and 8C, in clusters (G1 to G3) which have high drug response while having a high possibility of occurrence of a severe attack, or have a low possibility of occurrence of a severe attack, as a result of receiving treatment of Step 2 or Step 3 as shown in Table 3, more than half of patients do not have a severe attack within 12 months.

In the case of performing multivariate analysis or cluster analysis as described above, the measured levels may be normalized, in order to make the weights of the measured levels in the analysis uniform. The normalization is not particularly limited, and can be performed by methods known to those skilled in the art, for example, by normalizing measured levels so as to follow a log-normal distribution.

In one embodiment, a step of administering an attack therapeutic agent to an asthma patient determined to have a high exacerbation risk is included. By administering an attack therapeutic agent before a severe attack actually occurs, the possibility of a severe attack and the degree of attack of the patient can be reduced becomes high. Examples of the attack therapeutic agent include antibody drugs such as omalizumab and mepolizumab, oral steroids, and the like.

Also disclosed herein but not covered by the claims is a reagent kit used for the method of the claimed invention. That is, there is provided a reagent kit for distinguishing an asthma patient who is inadequately controlled by medium- to high-dose inhaled corticosteroid therapy and treatment with other asthma drugs, and with high exacerbation risk (hereinafter also referred to as "reagent kit"), comprising a first capture body that specifically binds to IL-17 for capturing IL-17, a second capture body that specifically binds to IL-17 for detecting IL-17, and a labeling substance. When the labeling substance is an enzyme, the reagent kit may further contain a substrate for the enzyme.

In the kits provided herein, the forms of the first capture body, the second capture body, the labeling substance and the substrate are not particularly limited, and they may be a solid (for example, powder, crystal, freeze-dried product, etc.) or liquid (for example, solution, suspension, emulsion, etc.). In the kits provided herein, it is preferable that the first capture body, the second capture body, the labeling substance and the substrate be stored in separate containers or individually packaged. The details of the blood sample, the first capture body, the second capture body, the labeling substance and the substrate are the same as those described in the explanation of the method. In the kits provided herein, it is preferable that both the first capture body and the second capture body be anti-IL-17 antibodies. When both the first capture body and the second capture body are monoclonal antibodies, it is preferable that the epitopes are different from each other.

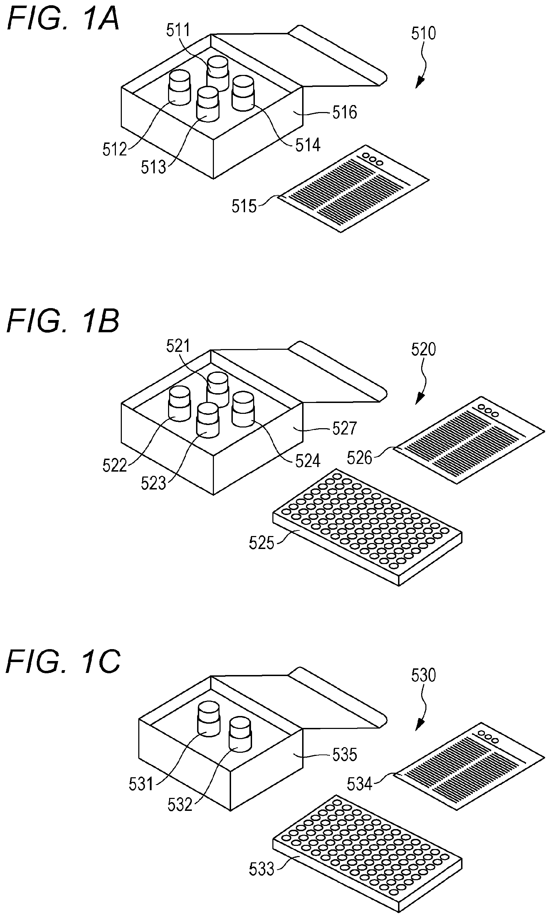

For example, a container storing the above-described various reagents may be packed in a box and provided to the user. This box may contain a package insert of the reagent kit. In this package insert, it is preferable to describe, for example, the constitution of the reagent kit, the protocol for measuring biomarker concentration in a blood sample, and the like. An example of the external appearance of the reagent kit is shown in Fig. 1A. In the figure, 510 denotes a reagent kit, 511 denotes a first container storing a first capture body, 512 denotes a second container storing a second capture body, 513 denotes a third container storing an enzyme as a labeling substance, 514 denotes a fourth container storing a substrate of the enzyme, 515 denotes a package insert, and 516 denotes a packing box.

The reagent kit disclosed but not claimed herein may further contain a solid phase for immobilizing the first capture body. In this case, it is preferable that the first capture body, the second capture body, the labeling substance, the substrate and the solid phase be stored in separate containers or individually packaged. An example of the external appearance of a reagent kit further containing a solid phase is shown in Fig. 1B. The details of the solid phase are the same as those described in the explanation of the method. In the figure, 520 denotes a reagent kit, 521 denotes a first container storing a first capture body, 522 denotes a second container storing a second capture body, 523 denotes a third container storing an enzyme as a labeling substance, 524 denotes a fourth container storing a substrate of the enzyme, 525 denotes a solid phase (96-well microplate), 526 denotes a package insert, and 527 denotes a packing box.

In a further example, the first capture body may be previously immobilized on a solid phase. The labeling substance may be previously bound to the second capture body. In this case, a reagent kit contains a solid phase on which a first capture body is immobilized, a second capture body to which a labeling substance is bound, and a substrate. An example of the external appearance of the reagent kit is shown in Fig. 1C. In the figure, 530 denotes a reagent kit, 531 denotes a first container storing a second capture body to which an enzyme as a labeling substance is bound, 532 denotes a second container storing a substrate of the enzyme, 533 denotes a solid phase (96-well microplate) on which a first capture body is immobilized, 534 denotes a package insert, and 535 denotes a packing box.

The reagent kit disclosed but not claimed herein may further comprise one or more capture body that specifically binds to at least one biomarker selected from the group consisting of IL-10, IL-4, IL-25, IL-9 and IFN-$\gamma$, and one or more capture body that specifically binds to the at least one biomarker for detecting the at least one biomarker, for acquiring a measured level of the at least one biomarker.

In addition to the various reagents described above, the reagent kit may appropriately contain a washing liquid of the solid phase, an enzyme reaction terminating agent, a calibrator, and the like.

The present disclosure also includes, but does not specifically claim the use of the above reagents for the manufacture of a reagent kit . That is, the present disclosure also relates to, but doers not specifically claim a use of a first capture body that specifically binds to IL-17 for capturing IL-17, a second capture body that specifically binds to IL-17 for detecting IL-17, and a labeling substance, for the manufacture of a reagent kit for predicting the possibility of occurrence of a severe attack of an asthma patient.

The present disclosure includes an apparatus suitable for acquiring data to distinguish an asthma patient who is inadequately controlled by medium- to high-dose inhaled corticosteroid therapy and treatment with other asthma drugs, and with high exacerbation risk. The present disclosure also includes a computer program product for making a computer acquire data to distinguish an asthma patient who is inadequately controlled by medium- to high-dose inhaled corticosteroid therapy and treatment with other asthma drugs, and with high exacerbation risk. The medium included in the computer program product may be a computer-readable medium in which a computer program is non-temporarily recorded.

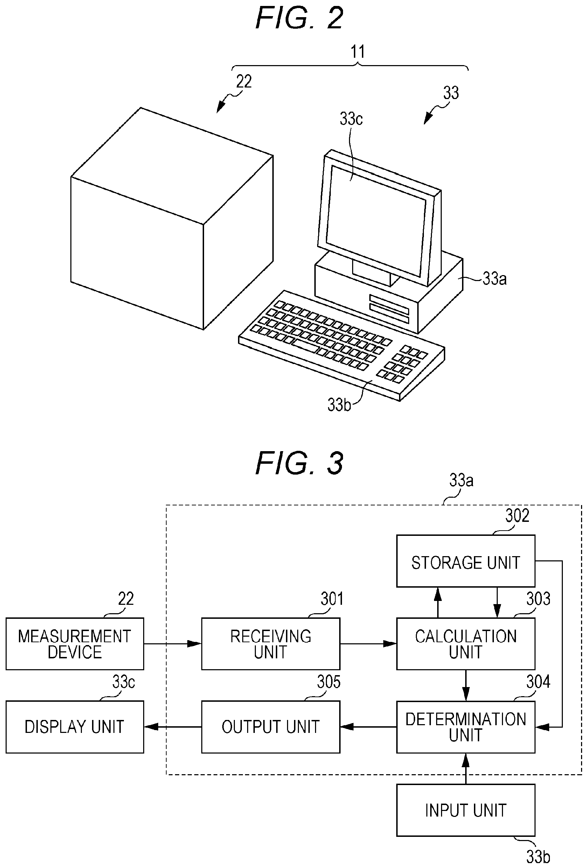

Hereinafter, an example of an apparatus suitable for implementing the above-described method of the present invention will be described with reference to the drawings. However, the present disclosure is not limited to only the embodiment shown in this example. Fig. 2 is a schematic diagram of an apparatus, for distinguishing an asthma patient who is inadequately controlled by medium- to high-dose inhaled corticosteroid therapy and treatment with other asthma drugs, and with high exacerbation risk. An apparatus 11 shown in Fig. 2 includes a measurement device 22, and a determination device 33 connected to the measurement device 22.

In the apparatus provided herein, the type of measurement device is not particularly limited, and it can be appropriately selected according to the method for measuring a biomarker. In the example shown in Fig. 2, the measurement device 22 is a plate reader capable of detecting a signal generated by an ELISA method using a labeled antibody. The plate reader is not particularly limited as long as it can detect a signal based on the labeling substance used for measurement of the biomarker, and it can be appropriately selected according to the type of the labeling substance. In the example described below, the signal is optical information such as a chemiluminescent signal or a fluorescence signal.

When the plate subjected to an antigen-antibody reaction is set in the measurement device 22, the measurement device 22 acquires optical information based on the labeled antibody specifically bound to the biomarker, and the measurement device 22 transmits the obtained optical information to the determination device 33. The optical information acquired by the measurement device 22 also includes information on a specimen of known concentration for calculation of the measured level of the biomarker and the like.

The determination device 33 includes a computer main body 33a, an input unit 33b, and a display unit 33c that displays specimen information, determination results, and the like. The determination device 33 receives the optical information from the measurement device 22. Then, a processor of the determination device 33 executes a program for acquiring data to distinguish an asthma patient who is inadequately controlled by medium- to high-dose inhaled corticosteroid therapy and treatment with other asthma drugs, and with high exacerbation risk, based on the optical information.

Fig. 3 is a block diagram showing software of the computer main body 33a of the apparatus 11 by functional blocks. As shown in Fig. 3, the determination device 33 includes a receiving unit 301, a storage unit 302, a calculation unit 303, a determination unit 304, and an output unit 305. The receiving unit 301 is communicably connected to the measurement device 22 via a network.

The receiving unit 301 receives the information transmitted from the measurement device 22. The storage unit 302 stores an equation for calculating a threshold level required for determination and a measured level of each biomarker, a processing program (for example, a program for executing multivariate analysis such as multiple logistic analysis and cluster analysis), and the like. Using the information acquired by the receiving unit 301, the calculation unit 303 calculates a measured level of biomarker or a value acquired by multivariate analysis, according to the equation stored in the storage unit 302. The determination unit 304 determines whether the measured level of biomarker or the value acquired by multivariate analysis calculated by the calculation unit 303 is equal to or greater than or less than the threshold value stored in the storage unit 302. The output unit 305 outputs the determination result of the determination unit 304 as data for distinguishing an asthma patient who is inadequately controlled by medium- to high-dose inhaled corticosteroid therapy and treatment with other asthma drugs, and with high exacerbation risk.

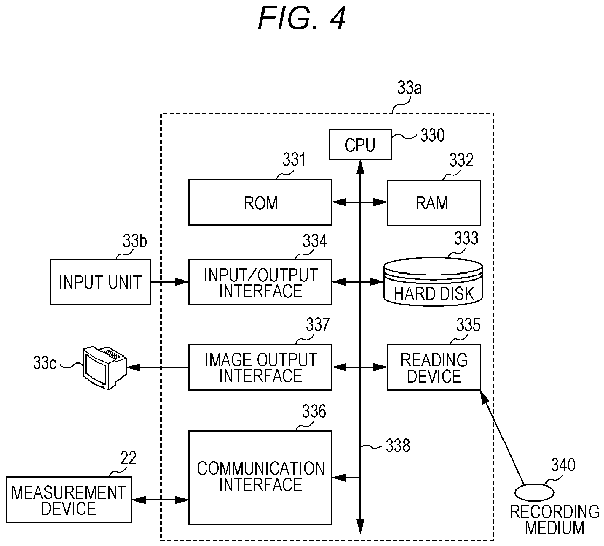

Fig. 4 is a block diagram showing a hardware configuration of the computer main body 33a shown in Fig. 3. As shown in Fig. 4, the computer main body 33a includes a CPU (Central Processing Unit) 330, a ROM (Read Only Memory) 331, a RAM (Random Access Memory) 332, a hard disk 333, an input/output interface 334, a reading device 335, a communication interface 336, and an image output interface 337. The CPU 330, the ROM 331, the RAM 332, the hard disk 333, the input/output interface 334, the reading device 335, the communication interface 336 and the image output interface 337 are data-communicably connected by a bus 338.

The CPU 330 can execute a computer program stored in the ROM 331 and a computer program loaded in the RAM 332. Each function shown in Fig. 3 is executed by the CPU 330 executing an application program. Accordingly, the determination device 33 functions as a device for acquiring data for distinguishing an asthma patient who is inadequately controlled by medium-to high-dose inhaled corticosteroid therapy and treatment with other asthma drugs, and with high exacerbation risk.

The ROM 331 is constituted of a mask ROM, a PROM, an EPROM, an EEPROM, and the like. In the ROM 331, a computer program executed by the CPU 330 and data used therefor are recorded.

The RAM 332 is constituted of an SRAM, a DRAM, and the like. The RAM 332 is used for reading the computer program recorded in the ROM 331 and the hard disk 333. The RAM 332 is also used as a work area of the CPU 330 when executing these computer programs.

The hard disk 333 has installed therein an operating system for making the CPU 330 execute, a computer program such as an application program (a computer program for distinguishing an asthma patient who is inadequately controlled by medium- to high-dose inhaled corticosteroid therapy and treatment with other asthma drugs, and with high exacerbation risk) and data used for executing the computer program.

The reading device 335 is constituted of a flexible disk drive, a CD-ROM drive, a DVD-ROM drive, and the like. The reading device 335 can read a computer program or data recorded on a portable recording medium 340.

p The input/output interface 334 is constituted of, for example, a serial interface such as USB, IEEE1394 or RS-232C, a parallel interface such as SCSI, IDE or IEEE1284, and an analog interface including a D/A converter, an A/D converter and the like. The input unit 33b such as a keyboard and a mouse is connected to the input/output interface 334. The operator can input various commands and data to the computer main body 33a through the input unit 33b.

The communication interface 336 is, for example, an Ethernet (registered trademark) interface or the like. The computer main body 33a can transmit print data to a printer or the like through the communication interface 336. The measurement device 22 can transmit measurement data (optical information) to the determination device 33 through the communication interface 336.

The image output interface 337 is connected to the display unit 33c constituted of an LCD, a CRT, and the like. Accordingly, the display unit 33c can output the video signal corresponding to the image data given from the CPU 330. The display unit 33c displays an image (screen) according to the inputted video signal.

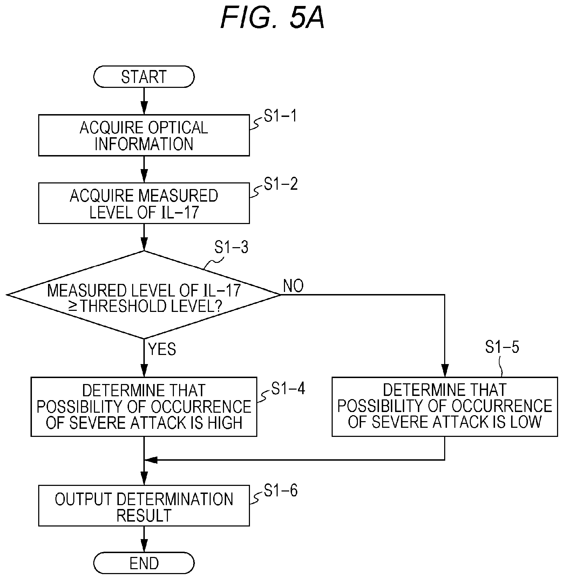

Next, a processing procedure for acquiring data to assist prediction of the possibility of occurrence of a severe attack of an asthma patient by the apparatus 11 will be described though it is not specifically claimed herein. Fig. 5A is an example of a flowchart for acquiring data on prediction of the possibility of occurrence of a severe attack of an asthma patient. A case where the measured level of IL-17 is acquired from the optical information based on the labeled antibody specifically bound to IL-17 and a determination is made using the obtained level will be described as an example.

First, in Step S1-1, the receiving unit 301 of the apparatus 11 receives optical information from the measurement device 22. Next, in Step S1-2, the calculation unit 303 calculates a measured level of IL-17, according to the equation for calculating the measured level of IL-17 stored in the storage unit 302, from the optical information received by the receiving unit 301.

Thereafter, in Step S1-3, the determination unit 304 determines whether the value calculated in Step S1-2 is less than or equal to or greater than the threshold value stored in the storage unit 302. When the calculated value is equal to or greater than the threshold value, the routine progresses to Step S1-4, and the determination unit 304 transmits to the output unit 305 a determination result indicating that the possibility of occurrence of a severe attack of an asthma patient is high. Also, when the calculated value is less than the threshold value, the routine progresses to Step S1-5, and the determination unit 304 transmits to the output unit 305 a determination result indicating that the possibility of occurrence of a severe attack of an asthma patient is low.

Then, in Step S1-6, the output unit 305 outputs the determination result. The output unit 305 displays it on the display unit 33c. Accordingly, the apparatus 11 can provide doctors and the like with data to assist prediction of the possibility of occurrence of a severe attack of an asthma patient.

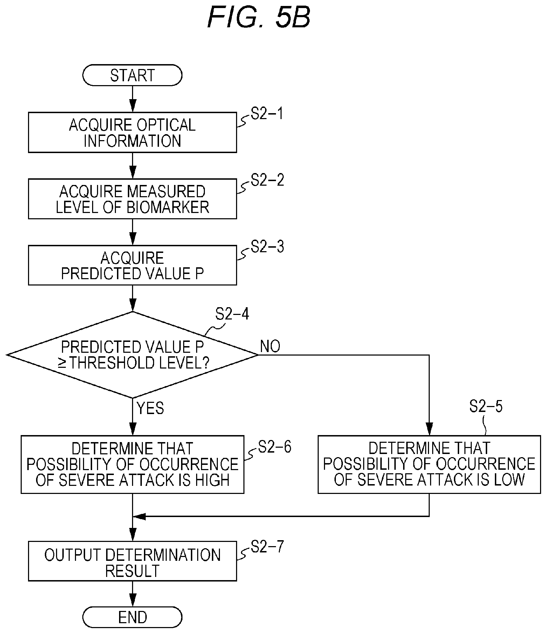

Fig. 5B is an example of a flowchart for acquiring data on prediction of the possibility of occurrence of a severe attack of an asthma patient, using multiple logistic analysis. A processing procedure for acquiring data to assist prediction of the possibility of occurrence of a severe attack of an asthma patient by the apparatus 11 will be described though not claimed herein. Here, a case where a measured level of biomarker is acquired from optical information based on a labeled antibody specifically bound to the biomarker and predicted value P is acquired by substituting the obtained measured level into discriminant, and a determination is performed using the predicted value P will be described as an example.

First, in Step S2-1, the receiving unit 301 of the apparatus 11 receives optical information from the measurement device 22. Next, in Step S2-2, the calculation unit 303 calculates a measured level of biomarker, according to the equation for calculating the measured level of biomarker stored in the storage unit 302, from the optical information received by the receiving unit 301, and further, in Step S2-3, the predicted value P is calculated according to the discriminant stored in the storage unit 302.

Thereafter, in Step S2-4, the determination unit 304 determines whether the predicted value P calculated in Step S2-3 is less than or equal to or greater than the threshold value stored in the storage unit 302. When the predicted value P is equal to or greater than the threshold value, the routine progresses to Step S2-5, and the determination unit 304 transmits to the output unit 305 a determination result indicating that the possibility of occurrence of a severe attack of an asthma patient is high. Also, when the predicted value P is less than the threshold value, the routine progresses to Step S2-6, and the determination unit 304 transmits to the output unit 305 a determination result indicating that the possibility of occurrence of a severe attack of an asthma patient is low.

Then, in Step S2-7, the output unit 305 outputs the determination result. The output unit 305 displays it on the display unit 33c. Accordingly, the apparatus 11 can provide doctors and the like with data to assist prediction of the possibility of occurrence of a severe attack of an asthma patient.

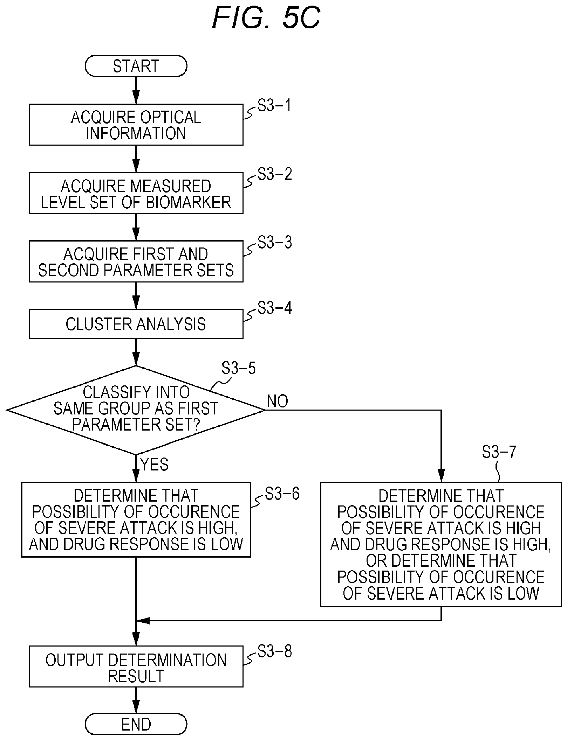

Fig. 5C is an example of a flowchart for acquiring data on distinguishing an asthma patient who is inadequately controlled by medium- to high-dose inhaled corticosteroid therapy and treatment with other asthma drugs, and with high exacerbation risk. A processing procedure for acquiring data to distinguishing an asthma patient who is inadequately controlled by medium- to high-dose inhaled corticosteroid therapy and treatment with other asthma drugs, and with high exacerbation risk by the apparatus 11 will be described. Here, a case where a parameter set of biomarkers is acquired from optical information based on a labeled antibody specifically bound to the biomarker and the like is acquired, and a determination is performed using the obtained parameter set will be described as an example. However, the present invention is not limited to this example.

In this embodiment, the storage unit 302 stores the first and second parameter sets, and the determination unit 304 performs cluster analysis to classify the asthma patient into the same group as one of the first and second parameter sets, and whether or not the exacerbation risk is high and whether or not the drug response is high are determined based on the classification result.

More specifically, first, in Step S3-1, the receiving unit 301 of the apparatus 11 receives optical information from the measurement device 22. Next, in Step S3-2, the calculation unit 303 calculates a measured level of biomarker, according to the equation for calculating the measured level of biomarker stored in the storage unit 302, from the optical information received by the receiving unit 301, and acquire a parameter set of biomarkers.

Thereafter, in Step S3-3, the determination unit 304 reads the first and second parameter sets stored in the storage unit 302. In Step S3-4, cluster analysis is performed using the parameter set of biomarkers acquired in Step S3-2 and the first and second parameter sets read in Step S3-3 to classify the asthma patient into the same group as one of the first and second parameter sets, and the routine proceeds to Step S3-5. When the asthma patient is classified into the same group as the first parameter set, the routine proceeds to Step S3-6, and the determination unit 304 transmits to the output unit 305 a determination result indicating that the exacerbation risk in the asthma patient is high, and the drug response Is low. On the other hand, when the asthma patient is not classified into the same group as the first parameter set, that is, when the asthma patient is classified into the same group as the second parameter set, the routine proceeds to Step S3-7, and the determination unit 304 transmits to the output section 305 a determination result indicating that the drug response is high while the exacerbation risk in the asthma patient is high, or the exacerbation risk in the asthma patient is low.

Then, in Step S3-8, the output unit 305 outputs the determination result. The output unit 305 displays it on the display unit 33c. Accordingly, the apparatus 11 can provide doctors and the like with data to distinguish an asthma patient who is inadequately controlled by medium- to high-dose inhaled corticosteroid therapy and treatment with other asthma drugs, and with high exacerbation risk.

Hereinafter, the present invention will be described in detail with reference to examples, but is not limited to these examples.

EXAMPLES

Example 1

(1) Sample

Serum samples of 77 asthma patients were obtained. The following clinical data was given to these samples. • History of severe attack within 12 months • Control status of asthma • ER delivery history within 12 months • Treatment step • Amount of therapeutic agent being administered • Respiratory function • Factor of atopy

(2) ELISA Measurement

ELISA measurement was performed as follows, using the serum sample obtained above, to measure the level of IL-17. ELISA measurement was performed by standard chemiluminescence ELISA, using anti-IL-17A antibody (eBio64CAP17 [eBioscience]) as a solid phase antibody and anti-IL-17A antibody (eBio64DEC17 [eBioscience]) as a detection antibody. That is, first, 50 $\mu$L of a solid phase antibody diluent (1 $\mu$g/mL solid phase antibody, 0.05% NaN$_3$, PBS PH7.4) was added to a 96 or 384-well ELISA plate, and the antibody was immobilized overnight at 4°C. The antibody was washed 3 times with washing buffer (0.05% Tween20, 0.05% NaN$_3$, PBS PH7.4) and blocked with 250 $\mu$L of blocking buffer (1% BSA, 0.5% Casein, 0.05% Tween20, 0.05% NaN$_3$, PBS PH7.4) at room temperature for 2 hours or more. 50

$\mu$L of serum diluted 4 times with ELISA Dilution Buffer (1% BSA, 0.5% Casein, 0.05% NaN$_3$, PBS PH7.4) was added thereto, and the mixture was reacted overnight at 4°C. The reactant was washed three times with washing buffer, and 50 $\mu$L of a labeled antibody solution (0.5 $\mu$g/mL labeled antibody, 1% BSA, 0.5% Casein, 0.05% NaN$_3$, PBS PH7.4) was added thereto. The reaction was performed at room temperature for 2 hours, and the reactant was washed three times with washing buffer. 50 $\mu$L of an alkaline phosphatase-labeled streptavidin solution (1:1000 diluted Streptavidin-Alkaline Phosphatase [R&D Systems AR001], 1% BSA, 0.5% Casein, 0.05% NaN$_3$, PBS PH7.4) was added thereto, and the mixture was reacted for 20 minutes. The reactant was washed three times with washing buffer and washed once with 250 $\mu$L of ALP activation buffer (20 mM Tris 10 mM MgCl pH9.8), and 100 $\mu$L of substrate (CDP-Star Ready-to-use Sapphire II Tropix) was added thereto, then luminescent signal was detected with a chemiluminescence detection device (FLUO star OPTIMA BMG LabTech).

(3) Analysis of ELISA measurement result

For each cytokine, a calibration curve was prepared from standard luminescent signals of known concentration. The luminescent signal obtained from the specimen was applied to the calibration curve to calculate the cytokine concentration in the serum. Analysis software MARS (BGM LABTECH) was used for analysis.

(4) Statistical analysis

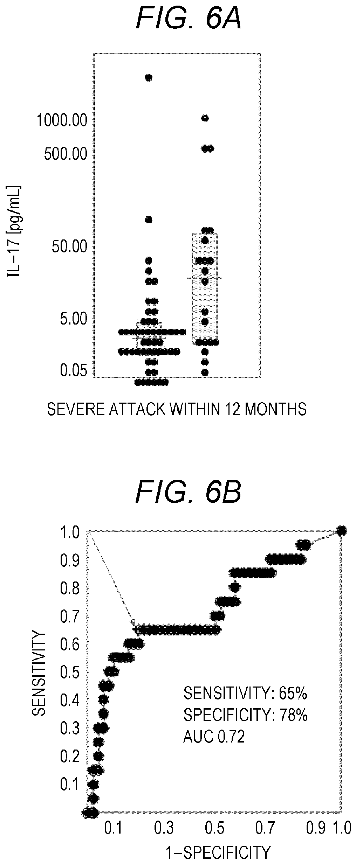

StatFlex version 6.0 (YUMIT) was used for statistical analysis. The data was divided into two groups of the patient group having a history of a severe attack within 12 months and the patient group having no history of a severe attack within 12 months, and the serum IL-17 concentration difference was tested. As a result, it was recognized that the serum IL-17 concentration was a significantly high value in the patient group having occurrence of a severe attack. In addition, the ROC curves were obtained, and the sensitivity and specificity were calculated by setting a cutoff of the occurrence and non-occurrence of a severe attack. The cutoff setting was set to the point closest to the point (sensitivity, 1-specificity) being (1, 0) in the ROC curve (indicated by an arrow in Fig. 6B).

The results are shown in Figs. 6A and 6B and Table 1. From these results, it was shown that the asthma patient group having a history of a severe attack and the asthma patient group having no history of a severe attack can be separated with significant difference, based on the blood IL-17 concentration.

[Table 1]

| Symptoms of exacerbation | p Value (welch's t-test) | IL-17 Cutoff value (pg/mL) | Sensitivity (%) | Specificity (%) | AUC |
|---|---|---|---|---|---|
| Presence or absence of occurrence of severe attack within 12 months | 0.011 | 5.29 | 65 | 80 | 0.72 |

Example 2

On the basis of the data obtained in Example 1, the patients were divided into a group of patients (20 subjects) with a higher value and a group of patients (57 subjects) with a lower value of the concentration of IL-17 than the 75 percentile of the examined asthma patients, and contrasted with the situation of exacerbation. The results are shown in Table 2 below. As shown in Table 2, in asthma patients having no history of a severe attack within 12 months, the IL-17 level was low in 43 out of 51 patients. In contrast, asthma patients having a history of a severe attack within 12 months, the IL-17 level was high in 12 out of 26 patients, and the percentage of patients with high IL-17 level increased. From these results, a significant difference (P value = 0.006) was found to exist in the blood IL-17A concentration between the asthma patient group having a history of a severe attack within 12 months and the asthma patient group having no history of a severe attack within 12 months.

[Table 2]

Clinical symptoms of asthma patients and concentration of IL-17A

| | IL-17A | | P value |
|---|---|---|---|
| | Low† | High‡ | |
| History of severe attack within 12 months | | | |
| Yes | 14 | 12 | 0.006 * |
| No | 43 | 8 | |

As described above, based on the results of Examples 1 and 2, it was found that it is possible to measure the level of IL-17 in the blood sample of the subject and acquire information on the possibility of occurrence of a severe attack of asthma in the subject based on the obtained measured level.

Example 3

In order to measure the amount of each cytokine other than IL-17, ELISA measurement was performed as follows, in the same manner as in Example 1.

- IL-10 ELISA measurement was performed by standard chemiluminescence ELISA, using anti-IL-10 antibody (JES3-9D7 [eBioscience]) as a solid phase antibody and anti-IL-10 antibody (JES3-12G8 [eBioscience]) as a detection antibody. That is, first, 50 $\mu$L of a solid phase antibody diluent (2 $\mu$g/mL solid phase antibody, 0.05% $NaN_3$, PBS PH7.4) was added to a 96 or 384-well ELISA plate, and the antibody was immobilized overnight at 4°C. The antibody was washed three times with washing buffer. The washed antibody was blocked with 250 $\mu$L of blocking buffer at room temperature for 2 hours or more. 50 $\mu$L of serum diluted 4 times with ELISA Dilution Buffer was added thereto, and the mixture was reacted overnight at 4°C. The reactant was washed three times with washing buffer, and 50 $\mu$L of a labeled antibody solution (0.5 $\mu$g/mL labeled antibody, 1% BSA, 0.5% Casein, 0.05% $NaN_3$, PBS PH7.4) was added thereto. The reaction was performed at room temperature for 2 hours, and the reactant was washed three times with washing buffer. 50 $\mu$L of an alkaline phosphatase-labeled streptavidin solution (1:1000 diluted Streptavidin-Alkaline Phosphatase [R&D Systems AR001], 1% BSA, 0.5% Casein, 0.05% $NaN_3$, PBS PH7.4) was added thereto, and the mixture was reacted for 20 minutes. The reactant was washed three times with washing buffer and washed once with 250 $\mu$L of ALP activation buffer (20 mM Tris 10 mM MgCl pH9.8), and 100 $\mu$L of substrate (CDP-Star Ready-to-use Sapphire II Tropix) was added thereto, then luminescent signal was detected with a chemiluminescence detection device (FLUO star OPTIMA BMG LabTech).
- IL-4 ELISA measurement was performed by standard chemiluminescence ELISA, using anti-IL-4 antibody (8D4-8 [Biolegend]) as a solid phase antibody and anti-IL-4 antibody (MP4-25D2 [Biolegend]) as a detection antibody. That is, first, 50 $\mu$L of a solid phase antibody diluent (2 $\mu$g/mL solid phase antibody, 0.05% $NaN_3$, PBS PH7.4) was added to a 96 or 384-well ELISA plate, and the antibody was immobilized overnight at 4°C. The antibody was washed three times with washing buffer. The washed antibody was blocked with 250 $\mu$L of blocking buffer at room temperature for 2 hours or more. 50 $\mu$L of serum diluted 4 times with ELISA Dilution Buffer (1% BSA, 0.05% $NaN_3$, PBS PH7.4) was added thereto, and the mixture was reacted overnight at 4°C. The reactant was washed three times with washing buffer, and 50 $\mu$L of a labeled antibody solution (0.5 $\mu$g/mL labeled antibody, 1% BSA, 0.05% $NaN_3$, PBS PH 7.4) was added thereto. The reaction was performed at room temperature for 2 hours, and the reactant was washed three times with washing buffer. 50 $\mu$L of an alkaline phosphatase-labeled streptavidin solution (1 : 1000 diluted Streptavidin-Alkaline Phosphatase [R&D Systems AR001], 1% BSA, 0.05% $NaN_3$, PBS PH7.4) was added thereto, and the mixture was reacted for 20 minutes. The reactant was washed three times with washing buffer and washed once with 250 $\mu$L of ALP activation buffer (20 mM Tris 10 mM MgCl pH9.8), and 100 $\mu$L of substrate (CDP-Star Ready-to-use Sapphire II Tropix) was added thereto, then luminescent signal was detected with a chemiluminescence detection device (FLUO star OPTIMA BMG LabTech).
- ELISA development kit (900-K234) manufactured by PeproTech, Inc. was used for IL-25 ELISA measurement.

First, 100 $\mu$L of antibody diluent (1 $\mu$g/mL solid phase antibody, PBS pH12.0) was added to an ELISA plate, and the antibody was immobilized overnight at 4 °C. The antibody was washed three times with washing buffer. The washed antibody was blocked with 250 $\mu$L of blocking buffer at room temperature for 2 hours or more. 100 $\mu$L of serum diluted 4 times with ELISA Dilution Buffer (1% BSA, 0.5% Casein, 0.05% $NaN_3$, PBS pH7.4) was added thereto, and the mixture was reacted overnight at 4°C. The reactant was washed three times with washing buffer, and 100 $\mu$L of a labeled antibody solution (0.25 $\mu$g/mL labeled antibody, 1% BSA, 0.5% Casein, 0.05% $NaN_3$, PBS) was added thereto. The reaction was performed at room temperature for 2 hours, and the reactant was washed three times with washing buffer. 100 $\mu$L of an alkaline phosphatase-labeled streptavidin solution (1:1000 diluted Streptavidin-Alkaline Phosphatase, 1% BSA, 0.05% $NaN_3$, PBS H7.4) was added thereto, and the mixture was reacted for 20 minutes. The reactant was washed three times with washing buffer and washed with 250 $\mu$L of ALP activation buffer, and 100 $\mu$L of substrate was added thereto, then luminescent signal was detected with a chemiluminescence detection device.

- ELISA development kit (900-K20) manufactured by PeproTech, Inc. was used for IL-9 ELISA measurement. First, 100 $\mu$L of antibody diluent (2 $\mu$g/mL solid phase antibody, PBS pH12.0) was added to an ELISA plate, and the antibody was immobilized overnight at 4°C. The antibody was washed three times with washing buffer. The washed antibody was blocked with 250 $\mu$L of blocking buffer at room temperature for 2 hours or more. 100 $\mu$L of serum diluted 4 times with ELISA Dilution Buffer (lowcross buffer [CANDOR]) was added thereto, and the mixture was reacted overnight at 4°C. The reactant was washed three times with washing buffer, and 100 μL of a labeled antibody solution (0.25 μg/mL labeled antibody, lowcross buffer [CANDOR]) was added thereto. The reaction was performed at room temperature for 2 hours, and the reactant was washed three times with washing buffer. 100 μL of an alkaline phosphatase-labeled streptavidin solution (1 : 1000 diluted lowcross buffer [CANDOR]) was added thereto, and the mixture was reacted for 20 minutes. The reactant was washed three times with washing buffer and washed with 250 μL of ALP activation buffer, and 100 μL of substrate was added thereto, then luminescent signal was detected with a chemiluminescence detection device.

- IFN-γ ELISA measurement was performed by standard chemiluminescence ELISA, using anti-IFN-γ antibody (NIB42 [Biolegend]) as a solid phase antibody and anti-IFN-γ antibody (4S.B3 [Biolegend]) as a detection antibody. That is, first, 50 μL of a solid phase antibody diluent (2 μg/mL solid phase antibody, 0.05% NaN$_3$, PBS PH7.4) was added to a 96 or 384-well ELISA plate, and the antibody was immobilized overnight at 4°C. The antibody was washed three times with washing buffer. The washed antibody was blocked with 250 μL of blocking buffer at room temperature for 2 hours or more. 50 μL of serum diluted 4 times with ELISA Dilution Buffer (1% BSA, 0.05% NaN$_3$, PBS PH7.4) was added thereto, and the mixture was reacted overnight at 4°C. The reactant was washed three times with washing buffer, and 50 μL of a labeled antibody solution (0.5 μg/mL labeled antibody, 1% BSA, 0.05% NaN$_3$, PBS PH 7.4) was added thereto. The reaction was performed at room temperature for 2 hours, and the reactant was washed three times with washing buffer. 50 μL of an alkaline phosphatase-labeled streptavidin solution (1 : 1000 diluted Streptavidin-Alkaline Phosphatase [R&D Systems AR001], 1% BSA, 0.05% NaN$_3$, PBS PH7.4) was added thereto, and the mixture was reacted for 20 minutes. The reactant was washed three times with washing buffer and washed once with 250 μL of ALP activation buffer (20 mM Tris 10 mM MgCl pH9.8), and 100 μL of substrate (CDP-Star Ready-to-use Sapphire II Tropix) was added thereto, then luminescent signal was detected with a chemiluminescence detection device (FLUO star OPTIMA BMG LabTech).

The serum concentrations of IL-17 and other cytokines obtained in the above examples were standardized, and cluster analysis was performed. More specifically, ELISA measurement results of each cytokine were subjected to logarithmic transformation and normalized, then standardized using the average value and the standard deviation. A hierarchical cluster analysis was performed with the nearest neighbor method • Euclidean distance, using the hclust function contained in R package stats. Thereafter, the patients were classified into clusters of G1 to G4, based on the phylogenetic trees obtained as a result of the hierarchical cluster analysis.

The results are shown in Fig. 7. As shown in Fig. 7, it was possible to distinguish patient clusters (G4) having generally high measured levels of IL-17, IL-10, IL-4, IL-25, IL-9 and IFN-γ. Figs. 8A to 8C show profiles of the patients as subjects of the cluster analysis of this example. The contents of the notation of symptoms described in the heat map of the lower part of Fig. 7 are as follows.

[Table 3]

| Indication of symptoms | Explanation | |
|---|---|---|
| ICS dose | • Dose of ICS (inhaled corticosteroid) that plays a central role in asthma treatment<br>• Therapies of Steps 1 to 4 are prepared according to the severity of patient in the guidelines. The dose of ICS is the highest in Step 4, and the lowest in Step 1. | |
| Daily medication step | • Step 1 | Low-dose inhaled corticosteroid |
| | • Step 2 | Low- to medium-dose inhaled corticosteroid and one of other asthma drugs |
| | • Step 3 | Combination of medium- to high-dose inhaled corticosteroid and other asthma drugs |
| Severe exacerbation within 12 month | • Occurred | Have severe attack within 12 months |
| | • Not occurred | No severe attack within 12 months |
| Control status | • Uncontrolled asthma | Inadequate asthma control |
| | • Controlled asthma | Good asthma control |

The percentages of severity for each cluster (G1 to G4) in each symptom are shown in Figs. 8A to 8C. In the group of patients distinguished as G4, 100% of the patients received medium- to high-dose inhaled corticosteroid therapy and treatment with other asthma drugs (Fig. 8A), but the patients were inadequately controlled by both treatment (Fig. 8B), and a severe attack occurred within 12 months in 75% of the patients (Fig. 8C).

Example 4

The serum concentrations of IL-17 and other cytokines obtained in the above examples were standardized, and cluster analysis of the concentration of IL-17 and the concentration of any other cytokine was performed. The patients were classified into clusters of G1 to G4, based on the phylogenetic trees obtained as a result of the cluster analysis.
The results are shown in Figs. 9A to 9E. As shown in Figs. 9A to 9E, G4 patients (refractory asthma patient population inadequately controlled even by medium- to high-dose inhaled corticosteroid therapy and treatment with other asthma drugs) could be distinguished, based on the measured levels of IL-17 and one other cytokine.

**Claims**

1. A method for distinguishing an asthma patient who is inadequately controlled by medium-to high-dose inhaled corticosteroid therapy and treatment with other asthma drugs, and with high exacerbation risk, the method comprising the steps of:

   measuring a biomarker in a blood sample of an asthma patient; and
   determining drug response and exacerbation risk in the asthma patient by comparing the measured levels of the biomarkers acquired in the measurement step with a predetermined threshold level corresponding to each biomarker, wherein it is determined that the exacerbation risk in the asthma patient is high, and the drug response is low when the measured level of IL-17 is equal to or greater than the predetermined threshold level and the measured level of any one of IL-10, IL-4, IL-25, IL-9 and IFN-γ is equal to or larger than the corresponding predetermined threshold level, wherein the drug response is the response to medium-to high dose inhaled corticosteroid therapy and treatment with other asthma drugs, and
   wherein the biomarker comprises IL-17 and at least one selected from the group consisting of IL-10, IL-4, IL-25, IL-9 and IFN-γ.

2. The method according to claim 1, wherein it is determined that the drug response is high while the exacerbation risk in the asthma patient is high, or the exacerbation risk in the asthma patient is low, when the measured level of IL-17 is equal to or greater than the predetermined threshold level and all the measured levels of IL-10, IL-4, IL-25, IL-9 and IFN-γ are less than the corresponding predetermined threshold level, or when the measured level of IL-17 is less than the predetermined threshold level.

3. A method for distinguishing an asthma patient who is inadequately controlled by medium-to high-dose inhaled corticosteroid therapy and treatment with other asthma drugs, and with high exacerbation risk, the method comprising the steps of:

   measuring IL-17 and at least one biomarker selected from the group consisting of IL-10, IL-4, IL-25, IL-9 and IFN-γ in a blood sample of an asthma patient to acquire a parameter set of biomarkers;
   classifying the asthma patient into the group with the highest correlation by performing cluster analysis using the parameter set of biomarkers, a first parameter set and a second parameter set; and
   determining the exacerbation risk in the asthma patient and the drug response, wherein it is determined that the asthma patient has a high exacerbation risk and a low drug response when classified into the same group as the first parameter set, and
   wherein it is determined that the asthma patient has a high drug response while having a high exacerbation risk, or that the asthma patient has a low exacerbation risk when classified into the same group as the second parameter set, wherein the drug response is the response to medium-to high dose inhaled corticosteroid therapy and treatment with other asthma drugs;
   wherein the parameter set of biomarkers comprises the measured level of IL-17 biomarker and the measured level of at least one biomarker selected from the group consisting of IL-10, IL-4, IL-25, IL-9 and IFN-γ,
   the first parameter set comprises the measured level of IL-17 and the measured level of the at least one biomarker selected from the group consisting of IL-10, IL-4, IL-25, IL-9 and IFN-γ in a blood sample of an asthma patient previously known to have an occurrence history of exacerbations and have low response to medium- to high-dose inhaled corticosteroid therapy and treatment with other asthma drugs, and
   the second parameter set comprises the measured level of IL-17 and the measured level of the at least one selected biomarker selected from the group consisting of IL-10, IL-4, IL-25, IL-9 and IFN-γ in a blood sample of an asthma patient previously known to have high response to medium- to high-dose inhaled corticosteroid therapy and treatment with other asthma drugs while having an occurrence history of exacerbations, or to have no occurrence history of exacerbations.

4. The method according to claim 3, wherein, in the classification step, the cluster analysis is performed by comparing the parameter set of biomarkers measured in the measurement step, the first parameter set, and the second parameter set.

5. The method according to any one of claims 1 to 4, wherein in the determination step, the risk of an exacerbation occurring in the asthma patient within 12 months is determined.

6. An apparatus for distinguishing an asthma patient who is inadequately controlled by medium- to high-dose inhaled corticosteroid therapy and treatment with other asthma drugs, and with high exacerbation risk, the apparatus comprising a measurement device for measuring a biomarker in a blood sample of an asthma patient and a determination device connected to the measurement device,
wherein the determination device comprises a computer containing a processor and a memory under control of the processor, wherein the memory stores a computer program for making the apparatus execute the method according to claim 1 or 2.

7. An apparatus for distinguishing an asthma patient who is inadequately controlled by medium- to high-dose inhaled corticosteroid therapy and treatment with other asthma drugs, and with high exacerbation risk, the apparatus comprising a measurement device for measuring a biomarker in a blood sample of an asthma patient and a determination device connected to the measurement device,

wherein the determination device comprises a computer containing a processor and a memory under control of the processor, wherein the memory stores a computer program for making the apparatus execute the method of claim 3 or 4,
wherein the memory stores the first parameter set and the second parameter set,
wherein the processor receives the parameter set of biomarkers measured in the measurement step, and reads the first parameter set and the second parameter set from the memory,

8. A computer program for distinguishing an asthma patient who is inadequately controlled by medium- to high-dose inhaled corticosteroid therapy and treatment with other asthma drugs, and with high exacerbation risk, which is recorded on a computer readable medium, the computer program comprising instructions to make the apparatus according to claim 6 or 7 execute the method of any one of claims 1 to 4.

**Patentansprüche**

1. Verfahren zum Erkennen eines Asthmapatienten, der durch mittel bis hoch dosierte Inhalationskortikosteroidtherapie und Behandlung mit anderen Asthmaarzneimitteln inadäquat kontrolliert ist, und mit hohem Exazerbationsrisiko, wobei das Verfahren die Schritte umfasst:

Messen eines Biomarkers in einer Blutprobe eines Asthmapatienten; und
Bestimmen einer Arzneimittelreaktion und eines Exazerbationsrisikos des Asthmapatienten durch Vergleichen der gemessenen Spiegel der Biomarker, die in dem Messschritt akquiriert wurden, mit einem vorbestimmten Schwellenwertspiegel, der jedem Biomarker entspricht, wobei bestimmt wird, dass das Exazerbationsrisiko des Asthmapatienten hoch ist und die Arzneimittelreaktion niedrig ist, wenn der gemessene Spiegel von IL-17 gleich dem vorbestimmten Schwellenwertspiegel oder größer als dieser ist, und der gemessene Spiegel von jedweden von IL-10, IL-4, IL-25, IL-9 und IFN-$\gamma$ gleich dem entsprechenden vorbestimmten Schwellenwertspiegel oder größer als dieser ist, wobei die Arzneimittelreaktion die Reaktion auf mittel bis hoch dosierte Inhalationskortikosteroidtherapie und Behandlung mit anderen Asthmaarzneimitteln ist, und wobei der Biomarker IL-17 und mindestens einen ausgewählt aus der Gruppe bestehend aus IL-10, IL-4, IL-25, IL-9 und IFN-$\gamma$ umfasst.

2. Verfahren nach Anspruch 1, wobei bestimmt wird, dass die Arzneimittelreaktion hoch ist, während das Exazerbationsrisiko des Asthmapatienten hoch ist, oder das Exazerbationsrisiko des Asthmapatienten niedrig ist, wenn der gemessene Spiegel von IL-17 gleich dem vorbestimmten Schwellenwertspiegel oder größer als dieser ist und alle der gemessenen Spiegel von IL-10, IL-4, IL-25, IL-9 und IFN-$\gamma$ kleiner als der entsprechende vorbestimmte Schwellenwertspiegel sind, oder wenn der gemessene Spiegel von IL-17 kleiner als der vorbestimmte Schwellenwertspiegel ist.

3. Verfahren zum Erkennen eines Asthmapatienten, der durch mittel bis hoch dosierte Inhalationskortikosteroidtherapie und Behandlung mit anderen Asthmaarzneimitteln inadäquat kontrolliert ist, und mit einem hohen Exazerbationsrisiko, wobei das Verfahren die Schritte umfasst:

Messen von IL-17 und mindestens einem Biomarker ausgewählt aus der Gruppe bestehend aus IL-10, IL-4, IL-25, IL-9 und IFN-$\gamma$ in einer Blutprobe eines Asthmapatienten, um einen Parametersatz von Biomarkern zu akquirieren;

Klassifizieren des Asthmapatienten in die Gruppe mit der höchsten Korrelation mittels Durchführen von Cluster-Analyse unter Verwendung des Parametersatzes von Biomarkern, eines ersten Parametersatzes und eines zweiten Parametersatzes; und

Bestimmen des Exazerbationsrisikos des Asthmapatienten und der Arzneimittelreaktion, wobei bestimmt wird, dass der Asthmapatient ein hohes Exazerbationsrisiko und eine niedrige Arzneimittelreaktion hat, wenn er in die gleiche Gruppe wie der erste Parametersatz klassifiziert wird, und

wobei bestimmt wird, dass der Asthmapatient eine hohe Arzneimittelreaktion hat, während er ein hohes Exazerbationsrisiko hat, oder dass der Asthmapatient ein niedriges Exazerbationsrisiko hat, wenn er in die gleiche Gruppe wie der zweite Parametersatz klassifiziert wird, wobei die Arzneimittelreaktion die Reaktion auf mittel bis hoch dosierte Inhalationskortikosteroidtherapie und Behandlung mit anderen Asthmaarzneimitteln ist;

wobei der Parametersatz der Biomarker den gemessenen Spiegel des Biomarkers IL-17 und den gemessenen Spiegel von mindestens einem Biomarker ausgewählt aus der Gruppe bestehend aus IL-10, IL-4, IL-25, IL-9 und IFN-$\gamma$ umfasst, der erste Parametersatz den gemessenen Spiegel von IL-17 und den gemessenen Spiegel des mindestens einen Biomarkers ausgewählt aus der Gruppe bestehend aus IL-10, IL-4, IL-25, IL-9 und IFN-$\gamma$ in einer Blutprobe eines Asthmapatienten umfasst, von dem vorbekannt ist, dass er eine Vorgeschichte des Auftretens von Exazerbationen hat und eine niedrige Reaktion auf mittel bis hoch dosierte Inhalationskortikosteroidtherapie und Behandlung mit anderen Asthmaarzneimitteln hat, und

der zweite Parametersatz den gemessenen Spiegel von IL-17 und den gemessenen Spiegel des mindestens einen ausgewählten Biomarkers ausgewählt aus der Gruppe bestehend aus IL-10, IL-4, IL-25, IL-9 und IFN-$\gamma$ in einer Blutprobe eines Asthmapatienten umfasst, von dem vorbekannt ist, dass er eine hohe Reaktion auf mittel bis hoch dosierte Inhalationskortikosteroidtherapie und Behandlung mit anderen Asthmaarzneimitteln hat, während er eine Vorgeschichte des Auftretens von Exazerbationen oder keine Vorgeschichte des Auftretens von Exazerbationen hat.

4. Verfahren nach Anspruch 3, wobei die Cluster-Analyse in dem Klassifizierungsschritt durchgeführt wird, indem der Parametersatz der Biomarker, die in dem Messschritt gemessen wurden, der erste Parametersatz und der zweite Parametersatz verglichen werden.

5. Verfahren nach einem der Ansprüche 1 bis 4, wobei in dem Bestimmungsschritt das Risiko bestimmt wird, dass bei dem Asthmapatienten eine Exazerbation innerhalb von 12 Monaten auftritt.

6. Vorrichtung zum Erkennen eines Asthmapatienten, der durch mittel bis hoch dosierte Inhalationskortikosteroidtherapie und Behandlung mit anderen Asthmaarzneimitteln inadäquat kontrolliert ist, und mit hohem Exazerbationsrisiko, wobei die Vorrichtung eine Messeinrichtung zum Messen eines Biomarkers in einer Blutprobe eines Asthmapatienten und eine Bestimmungseinrichtung umfasst, die mit der Messeinrichtung verbunden ist,

wobei die Bestimmungseinrichtung einen Computer umfasst, der einen Prozessor und einen Speicher unter Steuerung des Prozessors enthält, wobei der Speicher ein Computerprogramm speichert, um zu bewirken, dass die Vorrichtung das Verfahren gemäß Anspruch 1 oder 2 ausführt.

7. Vorrichtung zum Erkennen eines Asthmapatienten, der durch mittel bis hoch dosierte Inhalationskortikosteroidtherapie und Behandlung mit anderen Asthmaarzneimitteln inadäquat kontrolliert ist, und

mit hohem Exazerbationsrisiko, wobei die Vorrichtung eine Messeinrichtung zum Messen eines Biomarkers in einer Blutprobe eines Asthmapatienten und eine Bestimmungseinrichtung umfasst, die mit der Messeinrichtung verbunden ist,

wobei die Bestimmungseinrichtung einen Computer umfasst, der einen Prozessor und einen Speicher unter Steuerung des Prozessors enthält, wobei der Speicher ein Computerprogramm speichert, um zu bewirken, dass die Vorrichtung das Verfahren gemäß Anspruch 3 oder 4 ausführt,

wobei der Speicher den ersten Parametersatz und den zweiten Parametersatz speichert,

wobei der Prozessor den Parametersatz von Biomarkern empfängt, die in dem Messschritt gemessen wurden, und den ersten Parametersatz und den zweiten Parametersatz aus dem Speicher liest.

8. Computerprogramm zum Erkennen eines Asthmapatienten, der durch mittel bis hoch dosierte Inhalationskortikosteroidtherapie und Behandlung mit anderen Asthmaarzneimittel inadäquat kontrolliert ist, und mit hohem Exazerbationsrisko, das auf einem computerlesbaren Medium aufgezeichnet ist, wobei das Computerprogramm Anweisungen umfasst, um zu bewirken, dass die Vorrichtung gemäß Anspruch 6 oder 7 das Verfahren gemäß einem der Ansprüche 1 bis 4 ausführt.

**Revendications**

1. Procédé permettant de distinguer un patient asthmatique insuffisamment contrôlé par une corticothérapie inhalée à dose moyenne à élevée et un traitement par d'autres médicaments contre l'asthme, et présentant un risque élevé d'exacerbation, le procédé comprenant les étapes suivantes :

la mesure d'un biomarqueur dans un échantillon de sang d'un patient asthmatique ; et
la détermination de la réponse au médicament et du risque d'exacerbation chez le patient asthmatique en comparant les niveaux mesurés des biomarqueurs obtenus lors de l'étape de mesure avec un niveau seuil prédéterminé correspondant à chaque biomarqueur, dans lequel il est déterminé que le risque d'exacerbation chez le patient asthmatique est élevé, et que la réponse médicamenteuse est faible lorsque le niveau mesuré de IL-17 est égal ou supérieur au niveau seuil prédéterminé et que le niveau mesuré de l'une quelconque de IL-10, IL-4, IL-25, IL-9 et IFN-γ est égal ou supérieur au niveau seuil prédéterminé correspondant, la réponse médicamenteuse étant la réponse à une corticothérapie inhalée à dose moyenne à élevée et à un traitement par d'autres médicaments contre l'asthme, et
le biomarqueur comprenant l'IL-17 et au moins un marqueur choisi dans le groupe constitué par IL-10, IL-4, IL-25, IL-9 et IFN-γ.

2. Procédé selon la revendication 1, dans lequel il est déterminé que la réponse médicamenteuse est élevée alors que le risque d'exacerbation chez le patient asthmatique est élevé, ou que le risque d'exacerbation chez le patient asthmatique est faible, lorsque le niveau mesuré de IL-17 est égal ou supérieur au niveau seuil prédéterminé et que tous les niveaux mesurés de IL-10, IL-4, IL-25, IL-9 et de IFN-γ sont inférieurs au niveau seuil prédéterminé correspondant, ou lorsque le niveau mesuré de IL-17 est inférieur au niveau seuil prédéterminé.

3. Procédé permettant de distinguer un patient asthmatique insuffisamment contrôlé par une corticothérapie inhalée à dose moyenne à élevée et un traitement par d'autres médicaments contre l'asthme, et présentant un risque élevé d'exacerbation, le procédé comprenant les étapes suivantes :

la mesure de IL-17 et d'au moins un biomarqueur choisi dans le groupe constitué par IL-10, IL-4, IL-25, IL-9 et IFN-γ dans un échantillon de sang d'un patient asthmatique afin d'obtenir un ensemble de paramètres de biomarqueurs ;
le classement du patient asthmatique dans le groupe présentant la corrélation la plus élevée en effectuant une analyse de groupes à l'aide de l'ensemble de paramètres de biomarqueurs, d'un premier ensemble de paramètres et d'un second ensemble de paramètres ; et
la détermination du risque d'exacerbation chez le patient asthmatique et de la réponse médicamenteuse, dans lequel il est déterminé que le patient asthmatique présente un risque d'exacerbation élevé et une faible réponse médicamenteuse lorsqu'il est classé dans le même groupe que le premier ensemble de paramètres, et
dans lequel il est déterminé que le patient asthmatique a une réponse médicamenteuse élevée tout en ayant un risque d'exacerbation élevé, ou que le patient asthmatique a un faible risque d'exacerbation lorsqu'il est classé dans le même groupe que le deuxième ensemble de paramètres, la réponse médicamenteuse étant la réponse à une corticothérapie inhalée à dose moyenne à élevée et à un traitement par d'autres médicaments contre l'asthme ;
l'ensemble de paramètres des biomarqueurs comprenant le niveau mesuré du biomarqueur IL-17 et le niveau mesuré d'au moins un biomarqueur choisi dans le groupe constitué par IL-10, IL-4, IL-25, IL-9 et IFN-γ,
le premier ensemble de paramètres comprenant le niveau mesuré de IL-17 et le niveau mesuré d'au moins un biomarqueur sélectionné dans le groupe constitué par IL-10, IL-4, IL-25, IL-9 et IFN-γ dans un échantillon de sang d'un patient asthmatique précédemment connu pour avoir des antécédents d'exacerbations et une faible réponse à une corticothérapie inhalée à dose moyenne à élevée et à un traitement par d'autres médicaments contre l'asthme, et
le deuxième ensemble de paramètres comprenant le niveau mesuré de IL-17 et le niveau mesuré de l'au moins un biomarqueur sélectionné dans le groupe constitué par IL-10, IL-4, IL-25, IL-9 et IFN-$\gamma$ dans un échantillon de sang d'un patient asthmatique précédemment connu pour avoir une réponse élevée à une corticothérapie inhalée à dose moyenne à élevée et à un traitement par d'autres médicaments contre l'asthme tout en ayant des antécédents d'exacerbations, ou n'ayant pas d'antécédents d'exacerbations.

4. Procédé selon la revendication 3, lors de l'étape de classification, l'analyse de groupes étant réalisée en comparant l'ensemble de paramètres des biomarqueurs mesurés lors de l'étape de mesure, le premier ensemble de paramètres et le second ensemble de paramètres.

5. Procédé selon l'une des revendications 1 à 4, lors de l'étape de détermination, le risque de survenue d'une exacerbation chez le patient asthmatique dans les 12 mois étant déterminé.

6. Appareil permettant de distinguer un patient asthmatique insuffisamment contrôlé par une corticothérapie inhalée à dose moyenne à élevée et un traitement par d'autres médicaments contre l'asthme, et présentant un risque élevé d'exacerbation, l'appareil comprenant un dispositif de mesure pour mesurer un biomarqueur dans un échantillon de sang d'un patient asthmatique et un dispositif de détermination connecté au dispositif de mesure, le dispositif de détermination comprenant un ordinateur contenant un processeur et une mémoire sous la commande du processeur, la mémoire stockant un programme informatique permettant à l'appareil d'exécuter le procédé selon la revendication 1 ou 2.

7. Appareil permettant de distinguer un patient asthmatique insuffisamment contrôlé par une corticothérapie inhalée à dose moyenne à élevée et un traitement par d'autres médicaments contre l'asthme, et présentant un risque élevé d'exacerbation, l'appareil comprenant un dispositif de mesure pour mesurer un biomarqueur dans un échantillon de sang d'un patient asthmatique et un dispositif de détermination connecté au dispositif de mesure,

le dispositif de détermination comprenant un ordinateur contenant un processeur et une mémoire sous la commande du processeur, la mémoire stockant un programme informatique pour amener l'appareil à exécuter le procédé selon la revendication 3 ou 4,
la mémoire stockant le premier ensemble de paramètres et le second ensemble de paramètres,
le processeur recevant l'ensemble de paramètres des biomarqueurs mesurés lors de l'étape de mesure, et lisant le premier ensemble de paramètres et le second ensemble de paramètres dans la mémoire.

8. Programme informatique permettant de distinguer un patient asthmatique insuffisamment contrôlé par une corticothérapie inhalée à dose moyenne à élevée et un traitement par d'autres médicaments contre l'asthme, et présentant un risque élevé d'exacerbation, enregistré sur un support lisible par ordinateur, le programme informatique comprenant des instructions pour que l'appareil selon la revendication 6 ou 7 exécute le procédé selon l'une quelconque des revendications 1 à 4.

*FIG. 1A*

511
512
513
514
510
516
515

*FIG. 1B*

521
522
523
524
520
527
526
525

*FIG. 1C*

531
532
530
535
534
533

## FIG. 2

## FIG. 3

## FIG. 4

## FIG. 5A

```
                    ┌──────────┐
                    │  START   │
                    └────┬─────┘
                         │
                         ▼
              ┌──────────────────────┐   S1-1
              │  ACQUIRE OPTICAL     │
              │     INFORMATION      │
              └──────────┬───────────┘
                         │
                         ▼
              ┌──────────────────────┐   S1-2
              │   ACQUIRE MEASURED   │
              │     LEVEL OF IL-17   │
              └──────────┬───────────┘
                         │          S1-3
                         ▼
                  ╱──────────────╲
                 ╱  MEASURED LEVEL ╲        NO
                ╱   OF IL-17       ╲──────────────┐
                ╲  ≧THRESHOLD LEVEL?╱             │
                 ╲                 ╱              │
                  ╲───────┬───────╱               │
                         │ YES                    │        S1-5
                         ▼                        ▼
        ┌────────────────────────────┐   ┌────────────────────────────┐
        │     DETERMINE THAT         │   │     DETERMINE THAT         │
        │  POSSIBILITY OF OCCURRENCE │   │  POSSIBILITY OF OCCURRENCE │
        │  OF SEVERE ATTACK IS HIGH  │   │  OF SEVERE ATTACK IS LOW   │
        └──────────┬─────────────────┘   └─────────────┬──────────────┘
              S1-4 │                                    │
                   ▼◄───────────────────────────────────┘
        ┌────────────────────────────┐   S1-6
        │   OUTPUT DETERMINATION     │
        │         RESULT             │
        └──────────┬─────────────────┘
                   │
                   ▼
              ┌──────────┐
              │   END    │
              └──────────┘
```

## FIG. 5B

```
                    ┌─────────────┐
                    │    START    │
                    └──────┬──────┘
                           ▼
              ┌─────────────────────┐  ⌇S2-1
              │   ACQUIRE OPTICAL   │
              │     INFORMATION     │
              └──────────┬──────────┘
                         ▼
              ┌─────────────────────┐  ⌇S2-2
              │  ACQUIRE MEASURED   │
              │  LEVEL OF BIOMARKER │
              └──────────┬──────────┘
                         ▼
              ┌─────────────────────┐  ⌇S2-3
              │      ACQUIRE        │
              │  PREDICTED VALUE P  │
              └──────────┬──────────┘
                         ▼
                 S2-4
              ◇─────────────────◇
             ╱ PREDICTED VALUE P  ╲      NO
            ◇  ≥ THRESHOLD LEVEL?   ◇──────────┐
             ╲                     ╱           │
              ◇─────────────────◇              │
                       │ YES                   │
                       ▼                       ▼
   ┌───────────────────────────┐   ┌───────────────────────────┐
   │      DETERMINE THAT       │   │      DETERMINE THAT       │
   │ POSSIBILITY OF OCCURRENCE │   │ POSSIBILITY OF OCCURRENCE │
   │ OF SEVERE ATTACK IS HIGH  │   │  OF SEVERE ATTACK IS LOW  │
   └─────────────┬─────────────┘   └─────────────┬─────────────┘
          ⌇S2-6  │                          S2-5 │
                 ▼◄──────────────────────────────┘
      ┌─────────────────────┐  ⌇S2-7
      │ OUTPUT DETERMINATION│
      │       RESULT        │
      └──────────┬──────────┘
                 ▼
          ┌─────────────┐
          │     END     │
          └─────────────┘
```

# FIG. 5C

START

ACQUIRE OPTICAL
INFORMATION —S3-1

ACQUIRE MEASURED
LEVEL SET OF BIOMARKER —S3-2

ACQUIRE FIRST AND
SECOND PARAMETER SETS —S3-3

CLUSTER ANALYSIS —S3-4

CLASSIFY INTO
SAME GROUP AS FIRST
PARAMETER SET? —S3-5

NO

YES

DETERMINE THAT
POSSIBILITY OF OCCURENCE
OF SEVERE ATTACK IS HIGH,
AND DRUG RESPONSE IS LOW —S3-6

DETERMINE THAT
POSSIBILITY OF OCCURENCE
OF SEVERE ATTACK IS HIGH
AND DRUG RESPONSE IS HIGH,
OR DETERMINE THAT
POSSIBILITY OF OCCURENCE
OF SEVERE ATTACK IS LOW —S3-7

OUTPUT DETERMINATION
RESULT —S3-8

END

## FIG. 6A

SEVERE ATTACK WITHIN 12 MONTHS

## FIG. 6B

SENSITIVITY: 65%
SPECIFICITY: 78%
AUC 0.72

EP 3 367 101 B1

FIG. 7

*FIG. 8A*

NS

☐ Step1
☐ Step2
▦ Step3

*FIG. 8B*

NS

▦ UNCONTROLLED ASTHMA
☐ CONTROLLED ASTHMA

*FIG. 8C*

NS

▦ OCCURRED
☐ NOT OCCURRED

FIG. 9A

FIG. 9B

FIG. 9C

FIG. 9D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MAKOTO KUTO et al.** IL-17A produced by $\alpha\beta$ T cells drives airway hyper-responsiveness in mice and enhances muse and human airway smooth muscle contraction. *Nature Medicine,* vol. 18 (4), 547-554
- **IOANA AGACHE et al.** Increased serum IL-17 is an independent risk factor for severe asthma. *Respir Med,* 2010, vol. 104 (8), 1131-1137
- **CHESNÉ et al.** IL-17 ins severe asthma. Where do we stand?. *Am. J. Resp. Crit. Care Med.,* 2014, vol. 190 (10), 1094-1101
- **AL-RAMLI et al.** TH17-associated cytokines (IL-17A and IL-17F) in severe asthma. *J. Allergy Clin. Immunol.,* 2009, vol. 123 (5), 1185-1187
- **BULLENS et al.** IL-17 mRNA in sputum of asthmatic patients: linking T cell driven inflammation and granulocyte influx?. *Respir Res,* 2006, vol. 7 (1), 135
- **NIRAV BHAKTA et al.** IL-17 and TH2-high ashtrma: Adding fuel to the fire?. *J. Allergy Clin. Immunol.,* 2014, vol. 134 (5), 1187-1188
- **HASEGAWA et al.** Increased serum IL-17A and Th2 cytokine levels in patients with severe uncontrolled asthma. *Eur Cytokine Netw,* 2017, vol. 28 (1), 8-18